# EUROPEAN PATENT APPLICATION

(11) **EP 2 107 594 A2**
(43) Date of publication of application: **07.10.2009**
(21) Application number: 09165288.3
(22) Date of filing: 05.06.2002
(51) Int. Cl.: H01J 49/04, H01J 49/16, H01J 49/40, H01J 49/42

(54) **Apparatus and method for detecting organic trace components**

(30) Priority: 06.06.2001 JP 2001170753; 06.06.2001 JP 2001170778; 06.06.2001 JP 2001170784; 29.06.2001 JP 2001198574; 06.11.2001 JP 2001340487; 06.11.2001 JP 2001340495; 22.02.2002 JP 2002045801
(62) Divisional of application: 02733305.3
(71) Applicant: Mitsubishi Heavy Industries, Ltd., Minato-ku Tokyo 108-8215 (JP)
(72) Inventor: Deguchi, Yoshihiro, Nagasaki-shi Nagasaki 851-0392 (JP); Hiraki, Akio, Nagasaki-shi Nagasaki 851-0392 (JP); Noda, Matsuhei, Nagasaki-shi Nagasaki 851-0392 (JP); Kubota, Takahiro, Nagasaki-shi Nagasaki 851-0392 (JP); Hori, Junichiro, Nagasaki-shi Nagasaki 851-0392 (JP); Dobashi, Shinsaku, Nagasaki-shi Nagasaki 851-0392 (JP); Fukuda, Norihiro, Nagasaki-shi Nagasaki 851-0392 (JP)
(74) Representative: HOFFMANN EITLE

(57) **Abstract**

The present invention provides an apparatus for detecting an organic trace component, and the detection apparatus is employed for detecting an organic halogenated substance contained in a gas. The detection apparatus includes a capillary column (54) (sample introduction means) for continuously introducing a collected sample (51) in the form of a leakage molecular beam (53) into a vacuum chamber (52); laser irradiation means (66) for irradiating the leakage molecular beam (53) with a laser beam (55) to thereby perform ionization; a convergence section (56) for converging molecules ionized through laser irradiation, the section including a plurality of ion electrodes; an ion trap (57) for selectively trapping the thus-converged molecules; and a time-of-flight mass spectrometer (60) including an ion detector (59) for detecting ions which are emitted at predetermined intervals and reflected by a reflectron (58).

## Description

### Technical Field

The present invention relates to apparatus and method for detecting organic trace components, such as PCBs and dioxins, in treatment equipment or in the environment.

### Background Art

In recent years, bans have been imposed on production and importation of PCBs (polychlorinated biphenyls: generic term for chlorinated biphenyl isomers), because or their strong toxicity. In Japan, production of PCBs started around 1954. However, the Kanemi Yusho case revealed the adverse effects of PCBs on living organisms and the environment, and in 1972, the Japanese government issued an order that production of PCB products must be stopped and PCB products must be recovered (obligation of secure storage).

A PCB is a compound produced by substituting 1 to 10 chlorine atoms for hydrogen atoms of biphenyl. Theoretically, PCBs include 209 isomers, which differ in the number and position of substituted chlorine atoms. At present, about 100 or more PCB isomers are commercially available as PCB products. PCB isomers exhibit different physical and chemical properties, different levels of stability in living organisms, and various environmental behaviors. Therefore, chemical analysis of PCBs is difficult. Additionally, PCBs cause various types of environmental pollution. PCBs are persistent organic pollutants, and are not easily decomposed in the environment. PCBs exhibit fat solubility and high bioconcentration factor. Furthermore, PCBs can migrate through the air, because of their semi-volatility. PCBs are also known to remain in an environment; e.g., in water or in living organisms.

Since PCBs are very stable in living organisms, they accumulate therein, and the thus-accumulated PCBs cause chronic intoxication (e.g., skin diseases and liver diseases), and exhibit carcinogenicity and reproductive and developmental toxicity.

Conventionally, PCBs have been widely used as insulating oil for, for example, transformers and capacitors. In order to detoxify PCBs contained therein, the present inventors have previously proposed a hydrothermal decomposition apparatus for detoxifying PCBs (see, for example, Japanese Patent Application Laid-Open (*kokai*) Nos. 11-253796 and 2000-126588). FIG. 32 schematically shows the structure of such a hydrothermal decomposition apparatus.

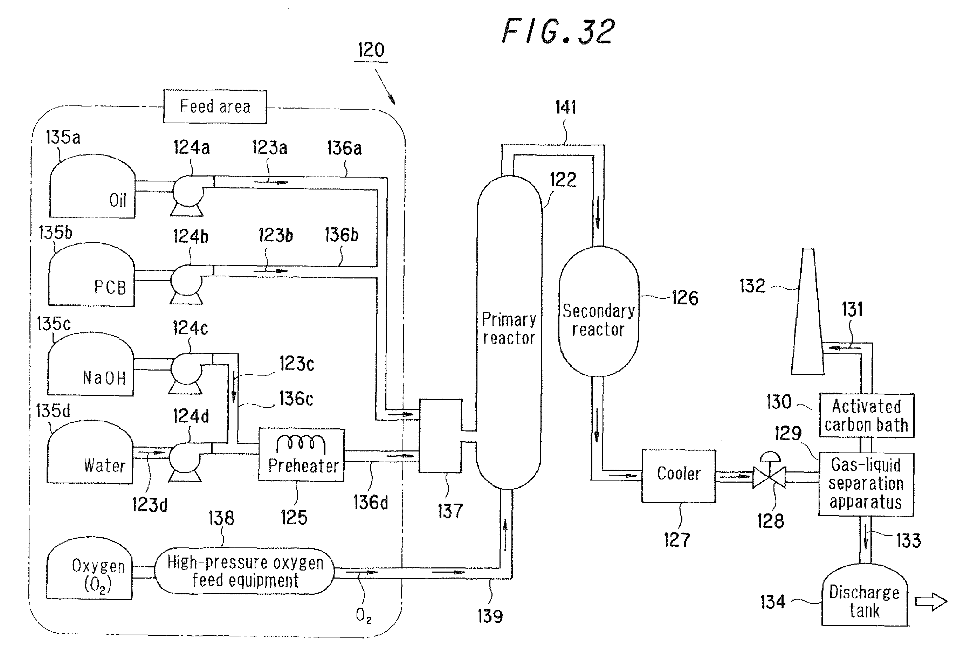

As shown in FIG. 32, a hydrothermal decomposition apparatus 120 includes a cylindrical primary reactor 122; pressurizing pumps 124a through 124d for pressurizing oil 123a, PCB 123b, NaOH 123c, and water 123d; a preheater 125 for preliminarily heating a liquid mixture of the NaOH 135c and the water 135d; a secondary reactor 126 having a spiral pipe; a cooler 127; and a pressure reduction valve 128. A gas-liquid separation apparatus 129 and an activated carbon bath 130 are provided downstream of the pressure reduction valve 128. Flue gas (CO₂) 131 is discharged from a chimney 132 to the outside, and a waste liquid (H₂O, NaCl) 133 is subjected to any treatment if desired. An oxygen feed pipe 139 is connected directly to the primary reactor 122.

In the aforementioned apparatus 120, the pressure of the interior of the primary reactor 122 is increased to 26 MPa by means of the pressurizing pumps 124. The liquid mixture 123 of PCB, H₂O, and NaOH is preliminarily heated to about 300°C by means of the preheater 125. Oxygen is fed into the primary reactor 122, and the temperature of the interior of the primary reactor 122 is increased to 380°C to 400°C by means of heat of a reaction generated in the reactor. In the primary reactor 122, PCBs undergo dechlorination reaction and oxidative degradation reaction, and are decomposed into NaCl, CO₂, and H₂O. Subsequently, the fluid discharged from the secondary reactor 126 is cooled to about 100°C in the cooler 127, and the pressure of the fluid is reduced to atmospheric pressure by means of the pressure reduction valve 128 provided downstream of the cooler 127. Thereafter, the fluid is separated into CO₂, steam, and treated water by means of the gas-liquid separation apparatus 129. The resultant CO₂ and steam are caused to pass through the activated carbon bath 130, and are discharged to the environment.

Through treatment of PCB-containing containers (e.g., transformers and capacitors) with the aforementioned processing apparatus, complete detoxification of the containers is achieved. During this process, quick monitoring of the concentration of PCBs in the facility is critical. Conventionally, PCBs have been sampled in the form of gas and concentrated in a liquid, and the PCB-containing liquid is subjected to analysis. However, measurement of the PCB concentration by means of such a technique requires some hours to some tens of hours, and thus quick monitoring of PCB concentration is not possible.

In view of the foregoing, conventionally, there has been proposed, as an apparatus for monitoring a trace amount of PCBs contained in a gas, a mass spectrometer incorporating a multi-photon ionization detector and a time-of-flight mass spectrometer (TOFMAS).

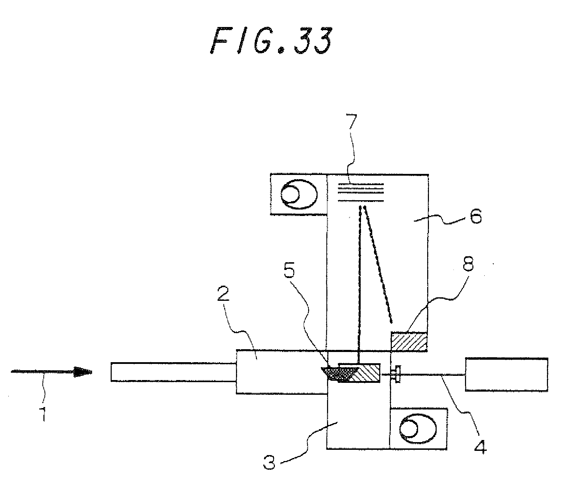

The conventional analysis apparatus will now be described with reference to FIG. 33.

As shown in FIG. 33, a sample gas 1 is fed as a supersonic free jet through a pulse nozzle 2 into a vacuum chamber 3. The free jet is cooled through adiabatic expansion. The vibrational and rotational level of the thus-cooled gas is lowered, thereby enhancing the wavelength selectivity of the gas. As a result, the gas efficiently absorbs a laser beam 4 (resonance multi-photon), and ionization efficiency of the gas is enhanced. Molecules contained in the thus-ionized gas are accelerated by means of an accelerating electrode 5, and an acceleration inversely proportional to the mass of the molecules is applied to the molecules. The thus-accelerated molecules fly through a flight tube 6. The molecules are reflected by a reflectron 7, and enter a detector 8. When the flight time of the molecules in the flight tube 6 is measured, the masses of particles consisting of the molecules are calculated. The concentration of PCBs (i.e., measurement target) can be obtained through comparison of the intensities of signals output from the detector 8.

Although the aforementioned analysis apparatus can detect a trace substance, the detection sensitivity of the apparatus is low, since the apparatus employs a laser having a pulse width in the order of nanoseconds.

In view of the foregoing, an object of the present invention is to provide an apparatus and method for detecting an organic trace component, which enable quick and highly-sensitive analysis when monitoring the concentration of PCBs contained in a gas.

In the aforementioned hydrothermal decomposition apparatus 120, various reaction parameters, including the feed amounts of various chemicals and PCBs (i.e., decomposition target), are controlled so as to decompose the PCBs completely.

Conventionally, decomposition treatment has been controlled on the basis of data obtained by measuring, for example, the amount of PCBs remaining in the treated liquid and properties of decomposition products produced during the course of decomposition treatment. However, such measurement requires some hours to one or two days.

Therefore, demand has arisen for efficient feedback control of the hydrothermal decomposition apparatus 120 during the course of decomposition treatment.

In view of the foregoing, another object of the present invention is to provide a method for controlling decomposition treatment of a toxic substance, which enables quick feedback control in an apparatus for decomposing toxic substances such as PCBs.

In use of the aforementioned apparatus for decomposing an organic halogenated substance, the concentration of PCBs in the working environment must be confirmed to be at a predetermined level or less at all times. Therefore, an apparatus for measuring a trace amount of PCBs must be calibrated at predetermined intervals so that the apparatus is operated properly.

In view of the foregoing, yet another object of the present invention is to provide an organic halogenated substance concentration correction apparatus which enables quick and highly-sensitive analysis when monitoring the concentration of a trace component such as PCBs.

### Disclosure of the Invention

The present invention includes the following inventions directed to an apparatus for detecting an organic trace component.

A first invention provides an apparatus for detecting an organic trace component comprising:
sample introduction means for continuously introducing a collected sample into a vacuum chamber;
laser irradiation means for irradiating the thus-introduced sample with a laser beam to thereby ionize the sample;
a convergence section for converging molecules that have been ionized through laser irradiation;
an ion trap for selectively trapping the thus-converged molecules; and
a time-of-flight mass spectrometer incorporating an ion detector for detecting ions which are emitted at predetermined intervals.

A second invention provides an apparatus for detecting an organic trace component according to the first invention, wherein the sample introduction means is a capillary column, and the tip of the capillary column projects into the convergence section.

A third invention provides an apparatus for detecting an organic trace component according to the first invention, wherein the capillary column is formed of quartz or stainless steel.

A fourth invention provides an apparatus for detecting an organic trace component according to the first invention, wherein the laser beam radiated from the laser irradiation means has a wavelength of 300 nm or less.

A fifth invention provides an apparatus for detecting an organic trace component according to the first invention, wherein the laser beam radiated from the laser irradiation means has a pulse width in the order of picoseconds.

A sixth invention provides an apparatus for detecting an organic trace component according to the first invention, wherein the laser beam radiated from the laser irradiation means has a pulse frequency of at least 1 MHz.

A seventh invention provides an apparatus for detecting an organic trace component according to the first invention, wherein the organic trace component is a PCB contained in a gas in treatment equipment where PCB decomposition treatment has been performed.

An eighth invention provides an apparatus for detecting an organic trace component according to the first invention, wherein the organic trace component is a PCB contained in a flue gas or waste liquid discharged from treatment equipment where PCB decomposition treatment has been performed.

A ninth invention provides an apparatus for detecting an organic trace component according to the first invention, wherein the laser beam radiated from the laser irradiation means has a wavelength of 300 nm or less, a pulse width of 1,000 picoseconds or less, and an energy density of 1 GW/cm² or less, and the organic trace component is detected while decomposition of the organic trace component is suppressed.

A tenth invention provides an apparatus for detecting an organic trace component according to the ninth invention, wherein the laser beam has an energy density of 1 to 0.01 GW/cm².

An eleventh invention provides an apparatus for detecting an organic trace component according to the ninth invention, wherein, when the organic trace component is a PCB having a small number of chlorine atoms (hereinafter the PCB may be referred to as "low-chlorine PCB"), the laser beam has a wavelength of 250 to 280 nm, a pulse width of 500 to 100 picoseconds, and an energy density of 1 to 0.01 GW/cm².

A twelfth invention provides an apparatus for detecting an organic trace component according to the ninth invention, wherein, when the organic trace component is a PCB having a large number of chlorine atoms (hereinafter the PCB may be referred to as "high-chlorine PCB"), the laser beam has a wavelength of 270 to 300 nm*, a pulse width of 500 to 1 picoseconds, and an energy density of 1 to 0.01 GW/cm².

A thirteenth invention provides an apparatus for detecting an organic trace component according to the first invention, wherein the laser beam has passed through a Raman cell.

A fourteenth invention provides an apparatus for detecting an organic trace component according to the thirteenth invention, wherein the Raman cell contains hydrogen.

A fifteenth invention provides an apparatus for detecting an organic trace component according to the first invention, wherein the ion trap comprises a first end cap electrode having a small hole through which the ionized molecules enter, a second end cap electrode having a small hole from which the trapped molecules are emitted, the first and second end cap electrode facing each other, and a high-frequency electrode for applying a high-frequency voltage to the ion trap; the voltage of the first end cap electrode is lower than that of the ion convergence section for converging the ionized molecules, and the voltage of the second end cap electrode is higher than that of the first end cap electrode; and the ionized molecules are trapped under application of the high-frequency voltage while the molecules within the ion trap are selectively decelerated.

A sixteenth invention provides an apparatus for detecting an organic trace component according to the fifteenth invention, wherein an inert gas is caused to flow within the ion trap.

A seventeenth invention provides an apparatus for detecting an organic trace component according to the fifteenth invention, wherein an ionization zone has a vacuum of 1 × 10⁻³ torr, the ion convergence section and the ion trap have a vacuum of 1 × 10⁻⁵ torr, and the time-of-flight mass spectrometer has a vacuum of 1 × 10⁻⁷ torr.

An eighteenth invention provides an apparatus for detecting an organic trace component according to the first invention, wherein the laser beam radiated from the laser irradiation means is repeatedly reflected such that the thus-reflected laser beams do not overlap one another within the ionization zone.

A nineteenth invention provides an apparatus for detecting an organic trace component according to the eighteenth invention, wherein the laser beam radiated from the laser irradiation means is repeatedly reflected by use of facing prisms such that the thus-reflected laser beams do not pass through the same path.

The present invention also includes the following inventions directed to a method for detecting an organic trace component.

A twentieth invention provides a method for detecting an organic trace component of a gas comprising: continuously introducing a collected sample into a vacuum chamber; irradiating the thus-introduced sample with a laser beam to thereby ionize the sample; selectively trapping, in an ion trap, molecules ionized through laser irradiation while converging the molecules; and detecting, by use of a time-of-flight mass spectrometer, ions which are emitted from the ion trap at predetermined intervals.

A twenty-first invention provides a method for detecting an organic trace component according to the twentieth invention, wherein the gas is a gas in treatment equipment where PCB decomposition treatment has been performed.

A twenty-second invention provides a method for detecting an organic trace component according to the twentieth invention, wherein the ion trap comprises a first end cap electrode having a small hole through which the ionized molecules enter, a second end cap electrode having a small hole from which the trapped molecules are emitted, the first and second end cap electrode facing each other, and a high-frequency electrode for applying a high-frequency voltage to the ion trap; the voltage of the first end cap electrode is lower than that of an ion convergence section for converging the ionized molecules, and the voltage of the second end cap electrode is higher than that of the first end cap electrode; and the ionized molecules are trapped under application of the high-frequency voltage while the molecules within the ion trap are selectively decelerated.

A twenty-third invention provides a method for detecting an organic trace component according to the twenty-second invention, wherein an inert gas is caused to flow within the ion trap, an ionization zone has a vacuum of 1 × 10⁻³ torr, the ion convergence section and the ion trap have a vacuum of 1 × 10⁻⁵ torr, and the time-of-flight mass spectrometer has a vacuum of 1 × 10⁻⁷ torr.

A twenty-fourth invention provides a method for detecting an organic trace component according to the twenty-first invention, wherein the gas is a gas in treatment equipment where PCB decomposition treatment has been performed.

A twenty-fifth invention provides a method for detecting an organic trace component according to the twenty-first invention, wherein the laser beam radiated from a laser irradiation means is repeatedly reflected such that the thus-reflected laser beams do not overlap one another within the ionization zone.

A twenty-sixth invention provides a method for detecting an organic trace component according to the twenty-fifth invention, wherein the laser beam radiated from the laser irradiation means is repeatedly reflected by use of facing prisms such that the thus-reflected laser beams do not pass through the same path.

The present invention also includes the following inventions directed to a method for controlling decomposition treatment of a toxic substance by use of the organic trace component detection apparatus.

A twenty-seventh invention provides a method for controlling decomposition treatment of a toxic substance comprising measuring, by use of the time-of-flight mass spectrometer as recited in the first invention, the concentration profile of a toxic substance and/or a product produced through decomposition of the toxic substance contained in a waste liquid, after decomposition treatment has been performed in a toxic substance decomposition apparatus comprising a reactor for decomposing a toxic substance; and optimizing conditions for decomposition treatment of a toxic substance on the basis of the thus-measured concentration profile of the toxic substance and/or the toxic substance decomposition product.

A twenty-eighth invention provides a method for controlling decomposition treatment of a toxic substance according to the twenty-seventh invention, wherein the toxic substance decomposition product is, for example, dichlorobenzene, a phthalate, a volatile organic compound, phenol, biphenyl, a derivative of benzene or biphenyl, an aldehyde, an organic acid, or an aromatic hydrocarbon.

The present invention also includes the following inventions directed to a toxic substance decomposition treatment system comprising the organic trace component detection apparatus.

A twenty-ninth invention provides a toxic substance decomposition treatment system comprising a hydrothermal oxidation-decomposition apparatus including a heated and pressurized reactor in which an organic halogenated substance is decomposed into, for example, sodium chloride (NaCl) and carbon dioxide (CO₂) through dechlorination and oxidation-decomposition in the presence of sodium carbonate (Na₂CO₃); an organic trace component detection apparatus as recited in the first invention for measuring the concentration of a toxic substance and/or a product produced through decomposition of the toxic substance contained in a waste liquid discharged from the hydrothermal oxidation-decomposition apparatus; and operation control means for controlling operation of the hydrothermal oxidation-decomposition apparatus on the basis of measurement results obtained from the organic trace component detection apparatus.

A thirtieth invention provides a toxic substance decomposition treatment system according to the twenty-ninth invention, wherein the hydrothermal oxidation-decomposition apparatus comprises a cylindrical primary reactor; a pressurizing pump for pressurizing oil or an organic solvent, a toxic substance, water (H₂O), and sodium hydroxide (NaOH); a preheater for preliminarily heating the water; a secondary reactor having a spiral pipe; a cooler for cooling a treated liquid discharged from the secondary reactor; gas-liquid separation means for subjecting the treated liquid to gas-liquid separation; and a pressure reduction valve.

A thirty-first invention provides a toxic substance decomposition treatment system according to the twenty-ninth invention, wherein the operation control means controls at least one selected from among heating of the toxic substance decomposition treatment system, pressurization of the system, the feed amount of a liquid for treating the toxic substance, the feed amount of an oxidizing agent, and the feed amount of sodium hydroxide (NaOH).

The present invention also includes the following inventions directed to an apparatus for simultaneously measuring the concentrations of gas samples obtained from a plurality of sampling points. The measuring apparatus incorporates the organic trace component detection apparatus.

A thirty-second invention provides an organic trace component measuring apparatus comprising an organic trace component detection apparatus as recited in the first invention; a plurality of sampling pipes for sampling a gas from sampling points provided on a gas path through which the gas passes; a valve provided on each of the sampling pipes; a combining pipe for connecting the sampling pipes to the laser irradiation means of the detection apparatus; gas suction means for circulating the gas, which is connected to the combining pipe; and cleanup means for discharging to the outside the gas remaining in a portion between the valve provided on each of the sampling pipes and the detection apparatus, the cleanup means being connected to the combining pipe.

A thirty-third invention provides an organic trace component measuring apparatus according to the thirty-secand invention, which further comprises a return pipe which is provided between the valve and a point at which each of the sampling pipes and the gas path are connected and which is connected to the gas path; and gas circulation means for circulating a gas, which is provided on the return pipe.

A thirty-fourth invention provides an organic trace component measuring apparatus according to the thirty-seccnd invention, wherein the gas suction means comprises a diaphragm pump connected to the combining pipe, and a valve provided between the combining pipe and the diaphragm pump.

A thirty-fifth invention provides an organic trace component measuring apparatus according to the thirty-second invention, wherein the cleanup means comprises a rotary scroll pump connected to the combining pipe, and a valve provided between the combining pipe and the rotary scroll pump.

A thirty-sixth invention provides an organic trace component measuring apparatus according to the thirty-second invention, wherein the valve is any valve selected from among a vacuum electromagnetic valve, an electric ball valve, and a bellows valve.

The present invention also includes the following inventions directed to an apparatus for calibrating the organic trace component measuring apparatus.

A thirty-seventh invention provides an organic halogenated substance concentration correction apparatus for calibrating the organic trace component detection apparatus as recited in the first invention, which comprises a standard container containing an organic halogenated substance of predetermined concentration; and a standard gas introduction tube for feeding into the standard container a purge gas for purging the organic halogenated substance, to thereby introduce into a mass spectrometer the organic halogenated substance accompanied by the purge gas.

A thirty-eighth invention provides an organic halogenated substance concentration correction apparatus according to the thirty-seventh invention, which further comprises temperature-maintaining means for maintaining the temperature of the standard container at a temperature 5 to 100 degrees higher than the temperature of an atmosphere surrounding the container.

A thirty-ninth invention provides an organic halogenated substance concentration correction apparatus according to the thirty-seventh invention, which further comprises temperature-maintaining means for maintaining the temperature of the standard gas introduction tube at 150°C or higher.

A fortieth invention provides an organic halogenated substance concentration correction apparatus according to the thirty-seventh invention, wherein a disk having a plurality of pores is provided in the standard container.

A forty-first invention provides an organic halogenated substance concentration correction apparatus according to the thirty-seventh invention, wherein the standard container is filled with glass fiber or beads.

A forty-second invention provides an organic halogenated substance concentration correction apparatus according to the fortieth invention, wherein a feed tube for feeding a purge gas is provided at the bottom of the standard container such that the outlet of the feed tube faces the bottom, and the fed purge gas is discharged from the upper portion of the container.

A forty-third invention provides an organic halogenated substance concentration correction apparatus according to the thirty-seventh invention, wherein the inner wall of the standard container is covered with a coating layer formed of polytetrafluoroethylene or silicon oxide.

A forty-fourth invention provides an organic halogenated substance concentration correction apparatus according to the thirty-seventh invention, wherein the standard container is removably provided.

A forty-fifth invention provides an organic halogenated substance concentration correction apparatus according to the forty-fourth invention, wherein the removable standard container is provided in a hermetic container.

A forty-sixth invention provides an organic halogenated substance concentration correction apparatus according to the forty-fifth invention, wherein a detection substance is fed into the standard container, and a sensor for detecting the detection substance is provided in the hermetic container.

A forty-seventh invention provides an organic halogenated substance concentration correction apparatus according to the forty-sixth invention, wherein the detection substance is hydrogen.

A forty-eighth invention provides an organic halogenated substance concentration correction apparatus according to the thirty-seventh invention, wherein the sample contains PCBs.

A forty-ninth invention provides a method for detecting an organic trace component comprising detecting an organic halogenated substance while correcting at predetermined intervals the concentration of the organic halogenated substance by use of the organic halogenated substance concentration correction apparatus as recited in the thirty-seventh invention.

A fiftieth invention provides a method for detecting an organic trace component according to the forty-ninth invention, wherein the sample contains PCBs, and the organic halogenated substance is a PCB.

### Brief Description of the Drawings

FIG. 1 is a schematic representation showing an apparatus for detecting an organic halogenated substance according to a first embodiment.
FIG. 2 shows results of measurement of low-chlorine PCBs.
FIG. 3 is a schematic representation showing a PCB concentration measuring system according to a second embodiment.
FIG. 4 shows the relation between the wavelength, pulse width, and energy density of a laser beam employed in a third embodiment.
FIG. 5 shows results of measurement of low-chlorine PCBs in the case where a laser beam having an energy density of 0.05 Gw/cm² is employed.
FIC. 6 shows results of measurement of low-chlorine PCBs in the case where a laser beam having an energy density of 0.5 GW/cm² is employed.
FIG. 7 is a schematic representation showing an apparatus for detecting an organic trace components according to a fourth embodiment.
FIG. 8 shows Raman effects of a laser beam which has passed through a Raman cell.
FIG. 9 shows the relation between the wavelength and energy of a laser beam under Raman effects.
FIG. 10 shows the relation between the wavelengths and ionization efficiencies of PCBs having 1 to 6 chlorine atoms.
FIG. 11 shows results of measurement of PCBs in the case where a laser beam which has passed through a Raman cell is employed.
FIG. 12 is a schematic representation showing an apparatus for detecting an organic trace component according to a fifth embodiment.
FIG. 13 is a schematic representation showing an ion trap.
FIG. 14 shows the relation between electric potential and ions approaching the center portion of the ion trap.
FIG. 15 is a schematic representation showing an apparatus for detecting an organic trace component according to a sixth embodiment.
FIG. 16 shows results of PCB analysis performed by use of the apparatus according to the sixth embodiment in which the vacuum is regulated to 1 × 10⁻⁵ torr.
FIG. 17 shows results of comparative PCB analysis by use of the apparatus according to the sixth embodiment in which the vacuum is regulated to 1 × 10⁻⁴ torr.
FIG. 18 is a schematic representation showing an apparatus for detecting an organic halogenated substance according to a seventh embodiment.
FIG. 19 is a schematic representation showing multiple reflection of a laser beam.
FIG. 20 is a schematic representation showing an optical apparatus for introducing a laser beam.
FIG. 21(A) is a side view of the apparatus shown in FIG. 20; and FIG 21(B) is a plan view of the apparatus shown in FIG. 20.
FIG. 22 is a schematic representation showing a PCB detoxification treatment system according to an eighth embodiment.
FIG. 23 is a schematic representation showing an organic halogenated substance decomposition treatment system according to a ninth embodiment.
FIG. 24 shows results of measurement of decomposition products.
FIG. 25 is a schematic representation showing the configuration of a multi-point measuring system according to a tenth embodiment.
FIG. 26 is a schematic representation showing an organic halogenated substance concentration correction apparatus according to an eleventh embodiment.
FIG. 27 is a schematic representation showing an essential portion of the organic halogenated substance concentration correction apparatus.
FIG. 28 is a schematic representation showing a standard container.
FIG. 29 is a schematic representation showing another standard container.
FIG. 30 is a schematic representation showing yet another standard container.
FIG. 31 is a chart showing results of measurement of PCBs after standard calibration is performed.
FIG. 32 is a schematic representation showing a hydrothermal decomposition apparatus.
FIG. 33 is a schematic representation showing a conventional measuring apparatus employing a laser beam.

### Best Mode for Carrying Out the Invention

In order to better illustrate the present invention, the best modes thereof will next be described with reference to the appended drawings. However, the present invention is not limited only to the embodiment described below.

### [First embodiment]

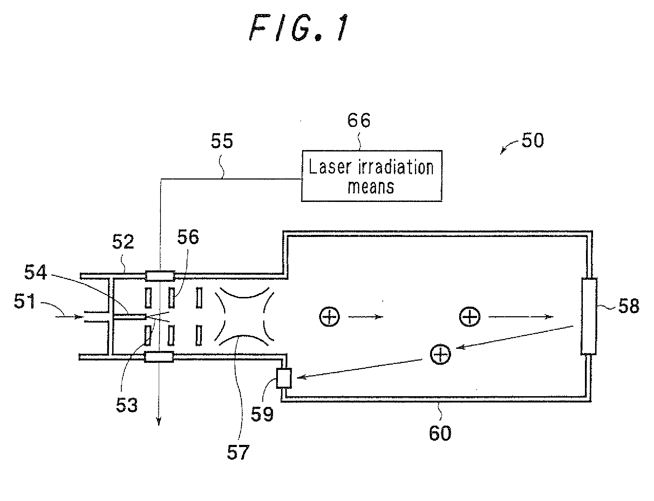

FIG. 1 is a schematic representation showing an apparatus for detecting an organic halogenated substance according to a first embodiment. An organic halogenated substance detection apparatus 50 of the present embodiment is employed for detecting an organic halogenated substance contained in a gas. As shown in FIG. 1, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58.

The concentration of PCBs (i.e., measurement target) can be obtained through comparison of the intensities of signals output from the detector 59.

The thus-obtained PCB concentration data may be sent to, for example, a monitor-control chamber, and to the outside by means of, for example, a monitoring apparatus (not illustrated) provided outside the detection apparatus 50.

Preferably, the capillary column 54 is provided such that the tip of the column projects into the ion convergence section 56. Specifically, the tip of the capillary column is made to be flush with an electrode constituting the ion convergence section 56 that is adjacent to the capillary column; or the capillary column is provided such that the tip thereof projects into the convergence section toward the ion trap by a certain length as measured from the electrode.

Preferably, the capillary column is formed of quartz or stainless steel. When the capillary column is formed of stainless steel, the leakage molecular beam can be regulated under application of voltage to the ion convergence section 56.

The diameter of the capillary column is preferably 1 mm or less. Preferably, the capillary column is provided such that the tip thereof is located about 3 mm away from the laser beam. Preferably, the outlet of the capillary is located a short distance away from a position at which the molecular beam is irradiated with the laser beam. However, when the distance between the outlet and the irradiation position is very small, the tip of the capillary column is broken by the laser beam. Therefore, preferably, the distance is decreased such that breakage of the tip does not occur; for example, the distance is decreased to about 1 to 2 mm, to thereby enhance ionization efficiency.

The pulse wavelength of the laser beam 55 radiated from the laser irradiation means 66 is 300 nm or less, preferably 266 ± 10 nm. When the pulse wavelength exceeds 300 nm, an organic halogenated substance (i.e., measurement target) fails to be ionized efficiently.

The pulse width of the laser beam 56 radiated from the laser irradiation means 66 is preferably in the order of picoseconds. When a laser beam having a pulse width in the order of nano (10⁻⁹)-seconds is employed, detection sensitivity is lowered.

Thus, when a laser beam having a pulse width in the order of pica (10⁻¹²) -seconds is employed, decomposition of PCB by the laser beam can be suppressed, and detection sensitivity can be enhanced.

Table 1 shows results of measurement of PCB detection sensitivities when laser beams having different pulse widths are employed.

This measurement employed a PCB sample containing PCBs having 1 to 4 chlorine atoms (hereinafter PCB having n chlorine atoms may be simply referred to as "n-Cl PCB") and predominantly containing 2-Cl PCB and 3-Cl PCB.

Signal intensities were measured by use of a laser beam having a pulse width of 100 picoseconds and a laser beam having a pulse width of 50 nanoseconds.

In the present embodiment, a pico-second laser (fixed wavelength: 266 nm) was employed for measurement.

**[Table 1] PCB detection sensitivities at different laser pulse widths**

| Measurement molecule | Signal intensity (mV) when a laser beam having a pulse width of 100 picoseconds is employed | Signal intensity (mV) when a laser beam having a pulse width of 50 nanoseconds is employed |
|---|---|---|
| 1-Cl PCB | 51.5 | 6.8 |
| 2-Cl PCB | 87.5 | 12.8 |
| 3-Cl PUB | 60.8 | 6.4 |
| 4-Cl PCB | 1.7 | 0.4 |
| PCB decomposition product | 50.4 | 123.4 |

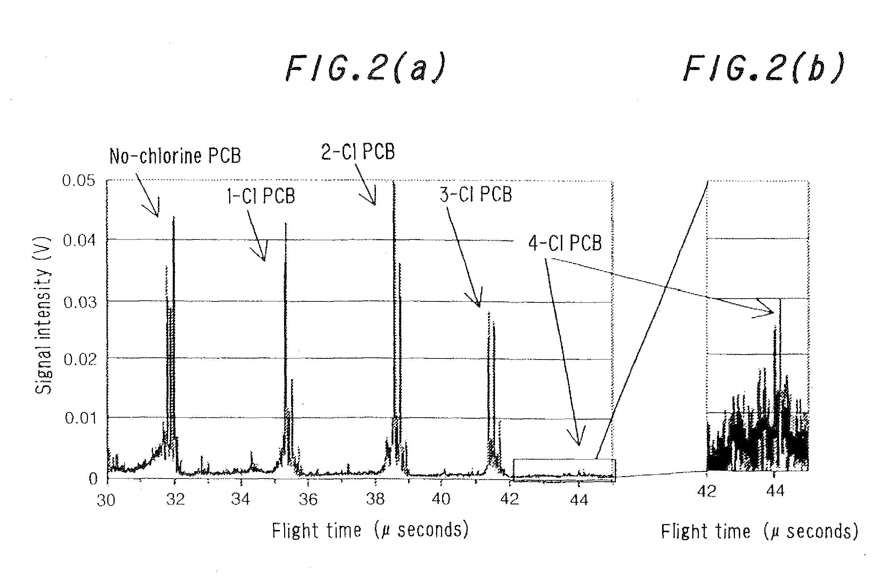

FIGs. 2 (a) and 2(b) are charts showing signals output from the ion detector which detected PCBs. In each of the charts, the horizontal axis represents flight time (seconds) and the vertical axis represents ion signal intensity (V). The signal corresponding to 4-Cl PCB is also shown in FIG. 2(b), which is an enlarged view of FIG. 2(a).

Through use of the aforementioned measuring apparatus, the concentration of PCBs remaining in, for example, a gas in PCB decomposition treatment equipment car waste liquid discharged from the equipment can be measured quickly and accurately. Monitoring of, for example, a treatment process can be performed on the basis of the measurement results.

When the concentration of PCBs remaining in waste liquid is measured, the waste liquid is introduced into the measuring apparatus, or the waste liquid is vaporized and the resultant vapor is introduced into the apparatus.

### [Second embodiment]

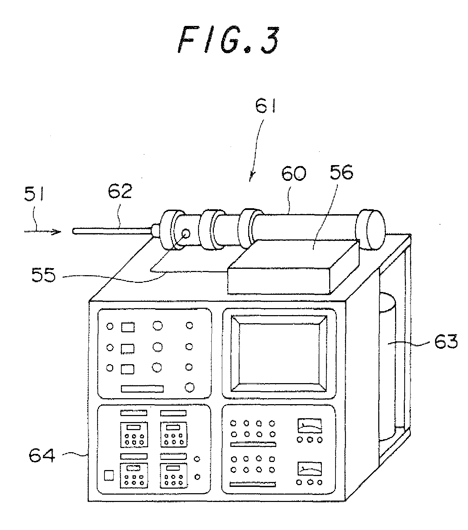

FIG. 3 is a schematic representation showing an apparatus for measuring the concentration of PCB contained in a gas.

As shown in FIG. 3, a PCB concentration measuring system 61 includes a gas sampling line 62 which is connected to the vacuum chamber 52 of the detection apparatus 50. As shown in FIG. 1, a sample is introduced as a leakage molecular beam into the vacuum chamber 52 through the line 62 and ionized by the laser beam 55 radiated from the laser irradiation means 66, and the resultant ions are detected by the time-of-flight mass spectrometer 60. In FIG. 3, reference numeral 63 denotes an evacuation apparatus for evacuating the vacuum chamber 52, and reference numeral 64 denotes a controller for controlling the aforementioned apparatuses.

Through use of the measuring system 61, quick and highly-sensitive PCB analysis can be performed; specifically, PCB can be detected at a sensitivity of 0.01 mg/Nm³ within one minute.

### [Third embodiment]

In the present embodiment, the laser irradiation conditions employed in the first embodiment are further specified.

A measuring apparatus according to the present embodiment has a structure similar to that of the apparatus of the first embodiment. Therefore, the apparatus of the present embodiment will be described with reference to FIG. 1.

In the first embodiment, the wavelength of the laser beam 55 radiated from the laser irradiation means 66 is 300 nm or less, preferably 266 ± 10 nm. In the present embodiment, when the organic halogenated substance (i.e., analysis target) is a low-chlorine PCB; i.e., a PCB having 1 to 3 chlorine atoms, more preferably, the wavelength of the laser beam is regulated to 250 to 280 nm.

Meanwhile, when the organic halogenated substance (i.e., analysis target) is a high-chlorine PCB; i.e., a PCB having at least 4 chlorine atoms, more preferably, the wavelength of the laser beam is regulated to 270 to 300 nm, for the following reason. When the number of chlorine atoms of PCB increases, the absorption wavelength of PCB shifts toward 300 nm.

In the present embodiment, the pulse width (laser pulse width) of the laser beam 55 radiated from the laser irradiation means 66 is preferably 500 pico (10⁻¹²)-seconds (ps) or less. When a laser beam having a pulse width in the order of nano (10⁻⁹)-seconds is employed, detection sensitivity is lowered.

In the present embodiment, the energy density (GW/cm²) of the laser beam 55 radiated from the laser irradiation means 66 is preferably 1 to 0.01 GW/cm², more preferably 0.05 to 0.01 GW/cm². When the laser energy density (GW/cm²) exceeds 1 GW/cm², the amount of PCB decomposition products increases.

As described above, in the present embodiment, the wavelength of the laser beam is regulated to 300 nm or less, the pulse width of the laser beam is regulated to 500 picoseconds or less, and the laser energy density is regulated to 1 GW/cm₂ or less. Thus, decomposition of PCB by the laser beam can be suppressed, and detection sensitivity can be greatly enhanced.

Particularly preferably, the energy density is regulated to 0.1 GW/am² or thereabouts.

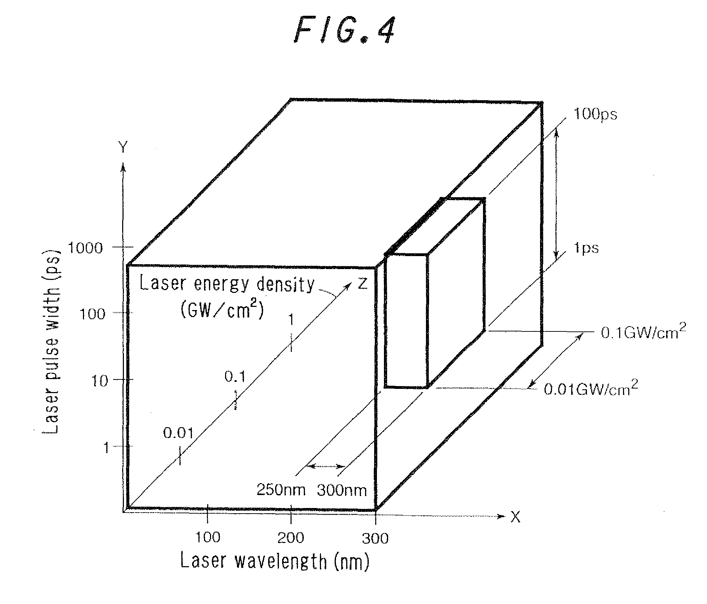

FIG. 4 shows the relation between the aforementioned wavelength, pulse width, and energy density.

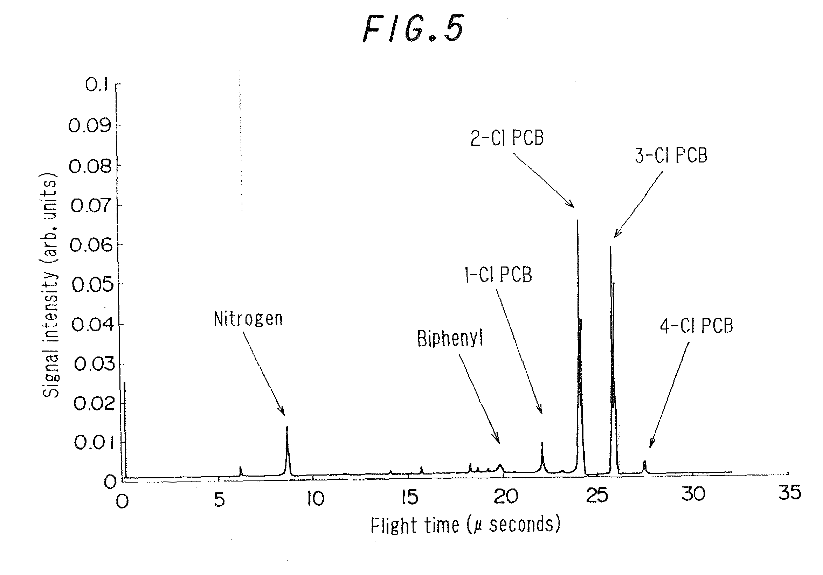

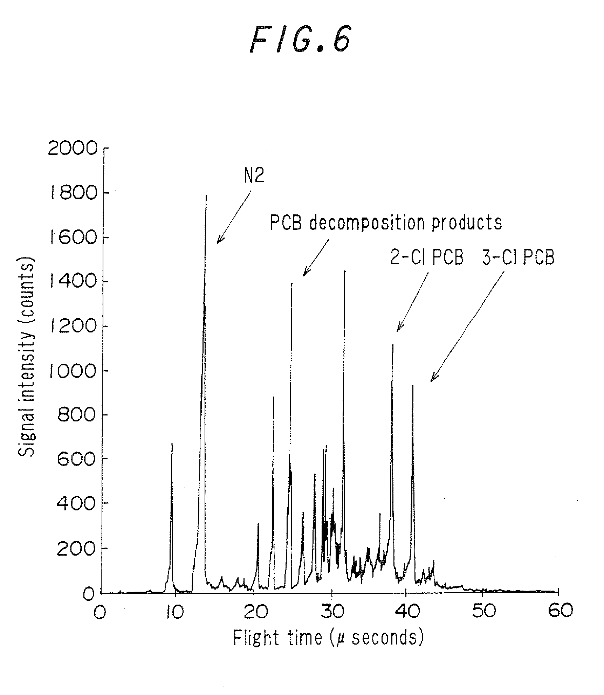

Mass spectra of a PCB standard sample and an N₂ gas sample were measured by use of the time-of-flight mass spectrometer. The results are shown in FIGS. 5 and 6. In this measurement, a laser beam having a pulse width of 100 picoseconds was employed. FIG. 5 shows measurement results for the case where the energy density of the laser beam is 0.05 GW/cm², and FIG. 6 shows measurement results for the case where the energy density of the laser beam is 0.5 GW/cm².

In this measurement, a PCB sample containing I- to 4-Cl PCBs and predominantly containing 2-Cl PCB and 3-Cl PCB was employed.

As shown in FIG. 5, in the case where the energy density of the laser beam is s 0.05 GW/cm², clear peaks corresponding to PCBs are obtained. In contrast, as shown in FIG. 6, in the case where the energy density of the laser beam is 0.5 GW/cm², large amounts of PCB decomposition products are generated, and clear peaks corresponding to PCBs are not obtained.

Through use of the aforementioned measuring apparatus, the concentration of PCB remaining in, for example, a gas in PCB decomposition treatment equipment can be measured quickly and accurately at high sensitivity. Monitoring of a treatment process can be performed on the basis of the measurement results.

### [Fourth embodiment]

In the present embodiment, the laser irradiation conditions in the first embodiment are further specified; specifically, a laser beam which has passed through a Raman cell is employed.

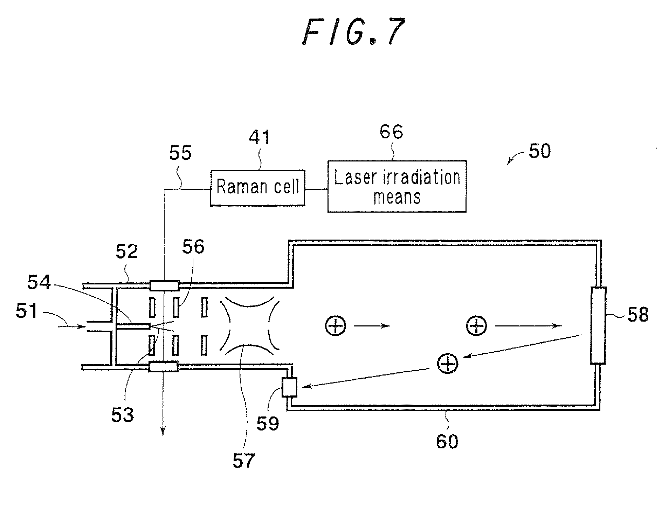

FIG. 7 is a schematic representation showing an organic trace component detection apparatus according to the present embodiment. An organic trace component detection apparatus 50 of the present embodiment is employed for detecting an organic trace component contained in waste liquid or flue gas. As shown in FIG. 7, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 which has passed through a Raman cell 41 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58.

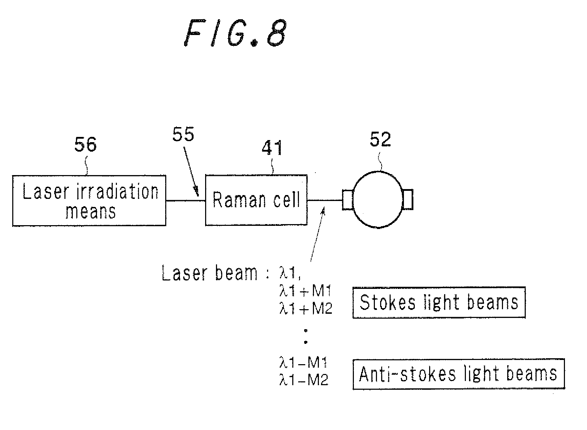

When the Raman cell 41 is provided, as shown in FIG. 8, the laser beam 55 (wavelength: λ₁) radiated from the laser irradiation means 66 is separated into Stokes light beams (wavelength: λ₁ + M₁, λ₁ + M₂ · · ·) and anti-Stokes light beams (wavelength: λ₁ - M₁, λ₁ - M₂ · · ·). Thus, a plurality of light beams having different wavelengths are obtained from the laser beam 55 having a single wavelength. Therefore, PCBs having different numbers of substituted chlorine atoms can be simultaneously excited by the thus-obtained light beams of different wavelengths.

Examples of the aforementioned Raman cell include a Raman cell containing a gas such as N₂, H₂, or CH₄ at high pressure (e.g., about 50 atm). When a laser beam of 266 nm is introduced into such a Raman cell, through interaction between molecules of a gas contained in the cell, a light beam having a specific wavelength is emitted from the cell; for example, a light beam of 283 nm is emitted in the case where the cell contains N₂, a light beam of 301 nm is emitted in the case where the cell contains H₂, or a light beam of 288 nm is emitted in the case where the cell contains CH₄.

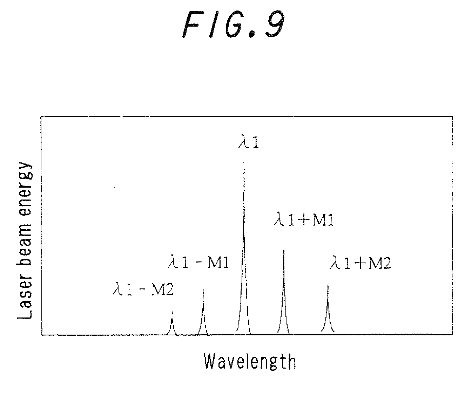

FIG. 9 shows the relation between the wavelengths and energies of light beams obtained when the laser beam 55 passes through the Raman cell 41.

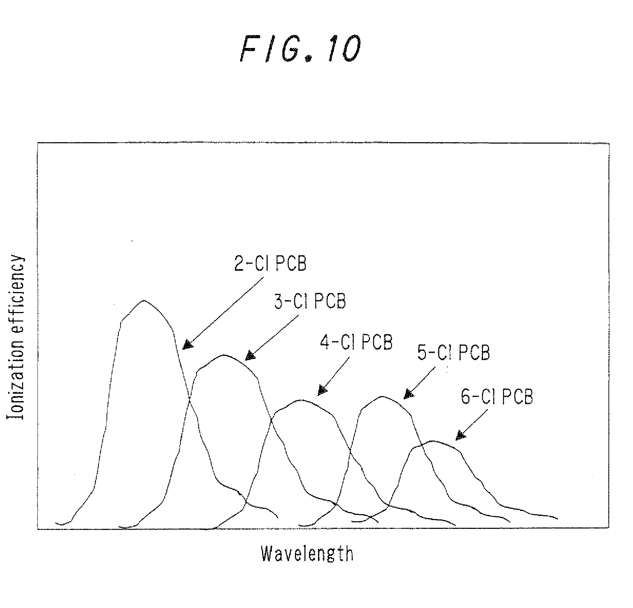

As shown in FIG. 10, when the number of substituted chlorine atoms of PCB increases, the absorption wavelength of the PCB shifts from a low level to a high level. Therefore, the concentrations of PCBs can be efficiently measured by use of a plurality of light beams having different wavelengths obtained from a laser beam which has passed through the Raman cell 41.

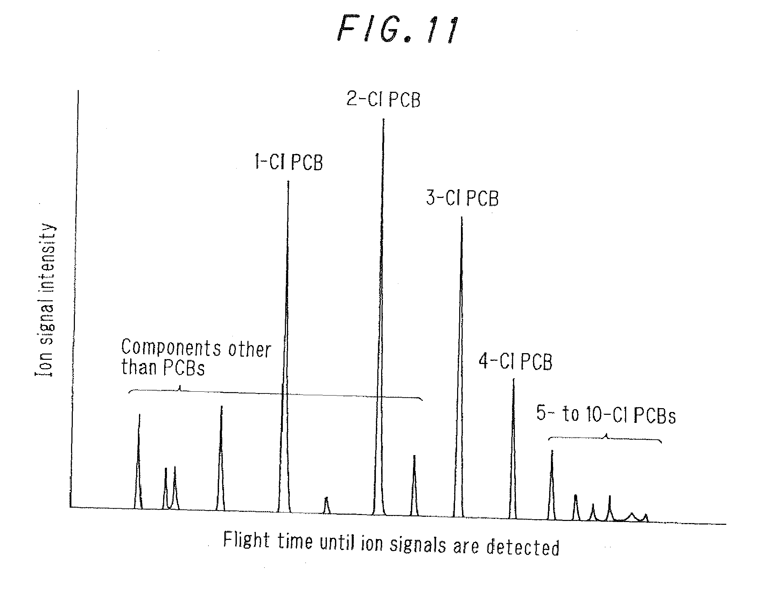

FIG. 11 shows results of measurement of PCBs by use of the apparatus shown in FIG. 7, in which a laser beam is separated into light beams of different wavelengths by means of the Raman cell. In the chart of FIG. 11, the horizontal axis represents flight time (seconds) and the vertical axis represents ion signal intensity (V).

The results show that efficient measurement can be performed by use of the apparatus shown in FIG. 7.

Through use of the aforementioned measuring apparatus, the concentration of PCB remaining in, for example, waste liquid discharged from PCB decomposition treatment equipment can be measured quickly and accurately. Monitoring of a treatment process can be performed on the basis of the measurement results.

### [Fifth embodiment]

In the present embodiment, the trapping conditions for molecules ionized through laser irradiation in the first embodiment are further specified.

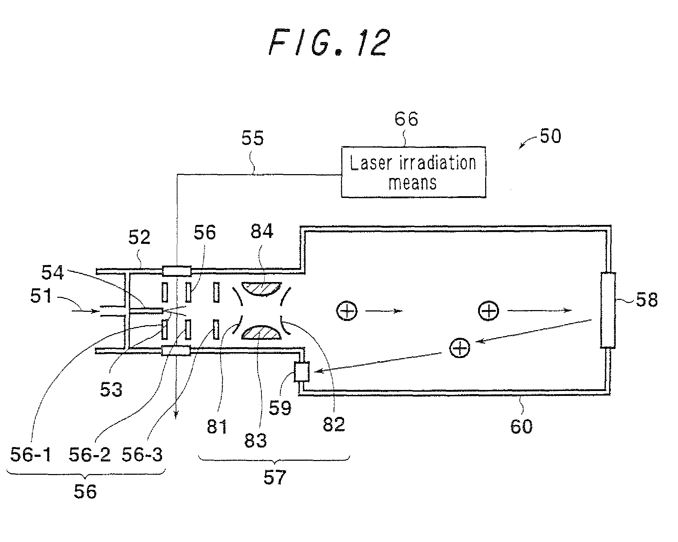

FIG. 12 is a schematic representation showing an organic trace component detection apparatus according to the present embodiment. An organic trace component detection apparatus 50 of the present embodiment is employed for detecting an organic trace component contained in waste liquid or flue gas. As shown in FIG. 12, the detection apparatus 50 includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes 56-1 to 56-3; an ion trap 57 for selectively trapping the thus-converged molecules; and a time-of-flight mass spectrometer 60 including an ion detector 59 for detecting ions which are emitted at predetermined intervals and reflected by a reflectron 58.

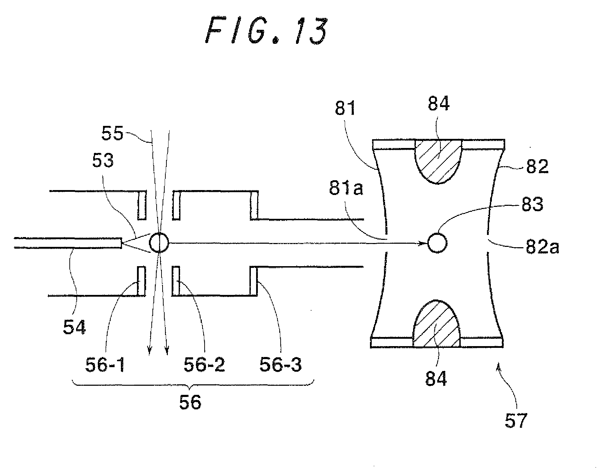

FIG. 13 is a schematic representation showing the ionization zone and the ion trap.

As shown in FIG. 13, the ion trap 57 includes a first end cap electrode 81 having a small hole &1a through which the ionized molecules enter; a second end cap electrode 82 having a small hole 82a from which the trapped molecules are emitted, the first and second end cap electrodes facing each other; and a high-frequency electrode 84 for applying high-frequency voltage to an ion trap zone 83.

The voltage of the first end cap electrode 81 is regulated to be lower than the voltage (e.g., 6 V) of the ion convergence section 56 for converging the ionized molecules; for example, the voltage of the electrode 81 is regulated to 0 V. The voltage of the second end cap electrode 82 is regulated to be higher than the voltage (e.g., 0 V) of the first end cap electrode 81; for example, the voltage of the electrode 82 is regulated to 12 V.

Since the voltage of the first end cap electrode 81 is regulated to be lower than the voltage (e.g., 6 V) of the ion convergence section 56 for converging the ionized molecules; for example, the voltage of the electrode 81 is regulated to 0 V, the ionized molecules are accelerated toward the first end cap electrode 81, and the molecules pass through the small hole 81a of the first end cap electrode 81 and efficiently enter the ion trap 57. The molecules are rapidly decelerated in the ion trap 57, since the voltage of the second end cap electrode 82 is regulated to be higher than the voltage (e.g., 0 V) of the first end cap electrode 81; for example, the voltage of the electrode 82 is regulated to 12 V. When high-frequency voltage is applied to the ion trap 57 by means of the high-frequency electrode 84, the molecules are rotated and trapped in the vicinity of the center of the ion trap 57.

After application of the high-frequency voltage by means of the high-frequency electrode 84 is stopped, when a high voltage (e.g., 400 V) is applied to the first end cap electrode 81 and a low voltage (e.g., -400 V) is applied to the second end cap electrode 82, the above trapped ions are emitted from the small hole 82a and are detected by the ion detector 59 provided in the time-of-flight mass spectrometer 60.

The concentration of a measurement target (e.g., PCBs) can be obtained through comparison of the intensities of signals output from the detector 59.

In the present invention, preferably, the voltage and frequency of the high-frequency electrode are regulated to 1,000 to 1,500 V and at least 1 MHz, respectively. In the case where PCBs are the targets of measurement, when the voltage and frequency are regulated to the above values, efficient trapping of ions is achieved in the ion trap zone.

No particular limitations are imposed on the voltage and frequency of the high-frequency electrode, since the voltage and frequency can be appropriately varied in accordance with the shape of the ion trap and the type of the measurement target, thereby optimizing conditions for ion trapping.

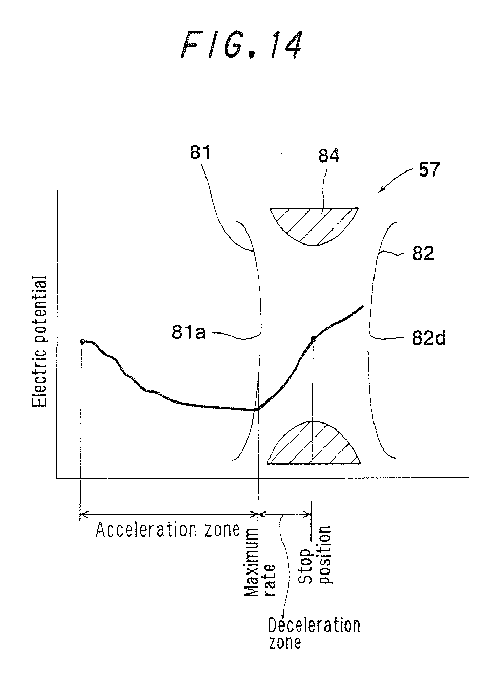

FIG. 14 shows the relation between electric potential and ions approaching the center portion of the ion trap in the case where the voltages of the first electrode 56-1, the second electrode 56-2, and the third electrode 56-3 are 6V, 6V, and 5 V, respectively; the voltage of the first end cap electrode 81 is 0 V; and the voltage of the second end cap electrode 82 is 12 V.

As shown in FIG. 14, ionized molecules are accelerated by means of the lensing effect of the convergence section 56, the velocity of the molecules becomes maximum at the first end cap electrode 81, and the thus-accelerated molecules pass through the small hole 81a of the first end cap electrode 81. The molecules are rapidly decelerated in the ion trap, since the voltage of the second end cap electrode 82 is 12V; i.e., the voltage of the electrode 82 is higher than that of the electrode 81. As a result, motion of the molecules stops in the vicinity of the center of the ion trap 57.

Motion of the trapped molecules stops in the vicinity of a position at which an electric potential is nearly equal to that of a position at which ionization of the molecules has been initiated. Therefore, the voltage of the second end cap electrode 82 may be determined so as to regulate the stop position of the trapped molecules.

Preferably, the voltage of the second end cap electrode 82 is regulated to become about twice that of the first electrode 56-1. In the present embodiment, the voltage of the first electrode 56-1 is regulated to 6V, and the voltage of the second end cap electrode 82 is regulated to 12 V.

The voltage of the second end cap electrode is not necessarily regulated to become twice that of the first electrode 56-1, and the voltage of the second end cap electrode may be optionally determined in accordance with the shape of the ion trap and the mass of molecules to be trapped.

As described above, in order to stop motion of the ionized sample in the ion trap, the voltage of the second end cap electrode 82 must be regulated to become higher than that of the first end cap electrode 81.

No particular limitations are imposed on the means for ionizing a sample. For example, the ionization means may be laser irradiation means for irradiating a sample with a laser beam to thereby ionize the sample. Alternatively, a sample may be ionized by use of, for example, an electron gun or plasma.

### [Sixth embodiment]

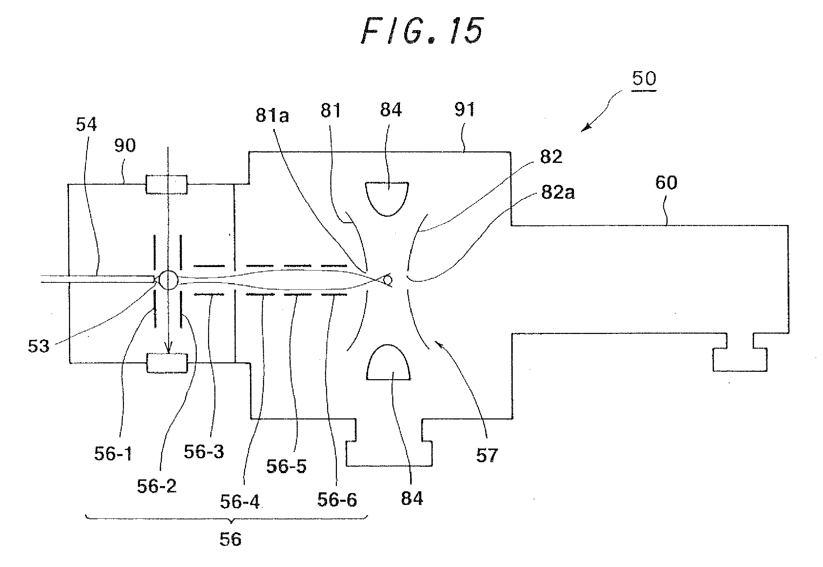

FIG. 15 is a schematic representation showing an organic trace component detection apparatus according to the present embodiment. An organic trace component detection apparatus 50 of the present embodiment is employed for detecting an organic trace component contained in waste liquid or flue gas. As shown in FIG. 15, the detection apparatus 50 includes an ionization zone 90 for ionizing a sample; a zone 91 including an ion convergence section 56 for converging ionized molecules and accelerating the molecules toward an ion trap 57, and the ion trap 57 for trapping ions; and a time-of-flight mass spectrometer 60. The ionization zone 90, the zone 91, and the spectrometer 60 are separated from one another by means of partition walls. The vacuum of the ionization zone 90 is regulated to 1 × 10⁻³ torr, the vacuum of the zone 91 including the ion convergence section and the ion trap is regulated to 1 × 10⁻⁵ torr, and the vacuum of the time-of-flight mass spectrometer 60 is regulated to 1 × 10⁻⁷ torr.

Components of the above apparatus that are similar to those employed in the apparatus shown in FIGS. 13 and 14 are denoted by common reference numerals, and repeated descriptions thereof are omitted.

In the present embodiment, the ionization zone 90 and the zone 91 including the ion convergence section and the ion trap are separated from each other. Therefore, unwanted gas is not introduced into the zone 91 including the ion convergence section and the ion trap, and thus efficient analysis is performed.

Since the vacuum of the zone 91 including the ion convergence section and the ion trap is regulated to as low as 1 × 10⁻⁵ torr, the amount of an inert gas can be reduced, and decomposition of a target substance which is readily decomposed can be prevented.

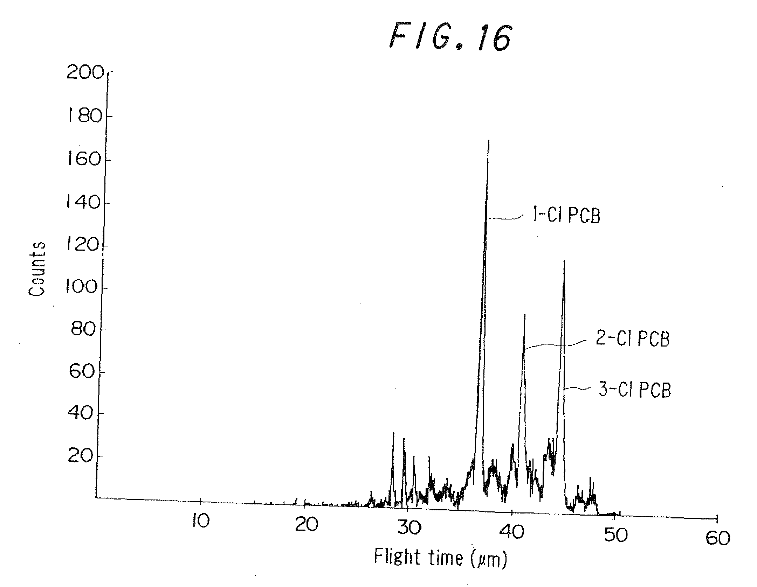

FIG. 16 shows results of measurement of PCBs in the case where the vacuum of the zone 91 including the ion convergence section and the ion trap was regulated to 1 × 10⁻⁵ torr. As shown in FIG. 16, clear peaks corresponding to 1-Cl PCB, 2-Cl PCB, and 3-Cl PCB were obtained.

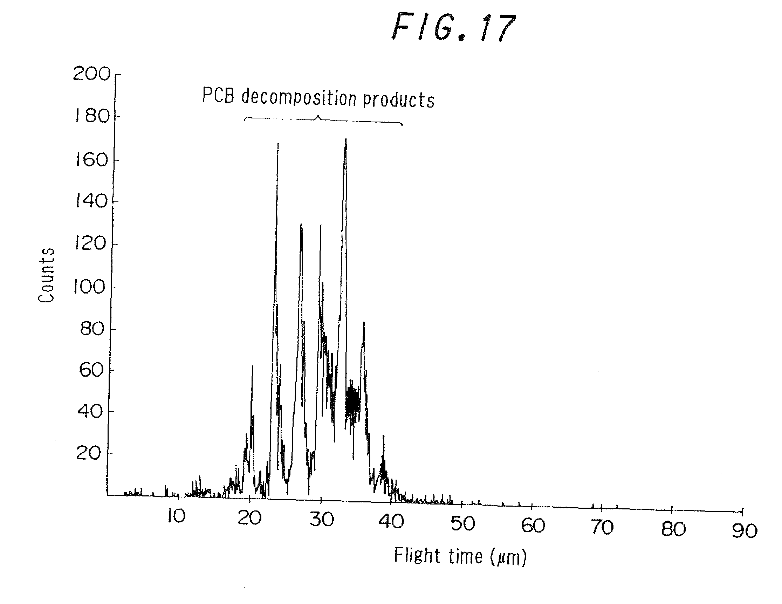

FIG. 17 shows results of measurement of PCBs in the case where the vacuum of the zone 91 including the ion convergence section and the ion trap was regulated to 1 × 10⁻⁴ torr.

As shown in FIG. 17, clear peaks corresponding to PCBs were not obtained; the peaks correspond to PCB decomposition products.

### [Seventh embodiment]

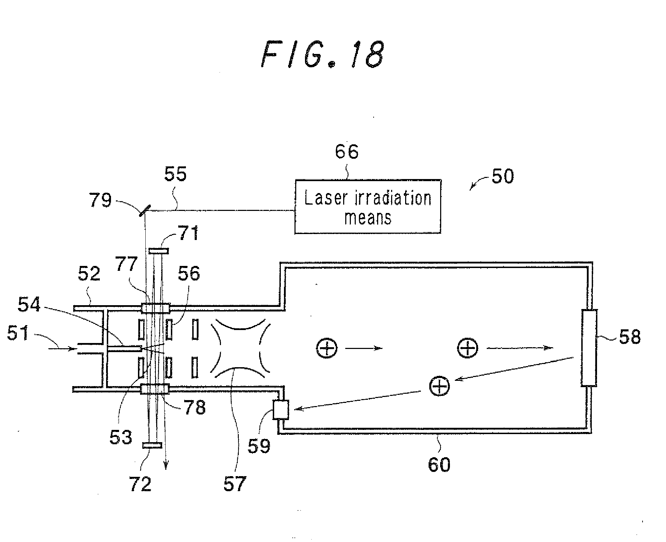

FIG. 18 is a schematic representation showing a laser-type measuring apparatus according to the present embodiment. As shown in FIG. 18, a laser-type measuring apparatus 50 of the present embodiment includes a capillary column 54 (sample introduction means) for continuously introducing a collected sample 51 in the form of a leakage molecular beam 53 into a vacuum chamber 52; laser irradiation means 66 for irradiating the leakage molecular beam 53 with a laser beam 55 to thereby perform ionization; a convergence section 56 for converging molecules ionized through laser irradiation, the section including a plurality of ion electrodes; an ion trap 57 for selectively trapping the thus-converged molecules; and a time-of-flight mass spectrometer 60 including an ion detector 59 for detecting ions which are emitted at predetermined intervals and reflected by a reflectron 58. The concentration of a measurement target can be obtained through comparison of the intensities of signals output from the detector 59.

In FIG. 18, reference numerals 77 and 78 denote lens windows, and reference numeral 79 denotes a reflection mirror.

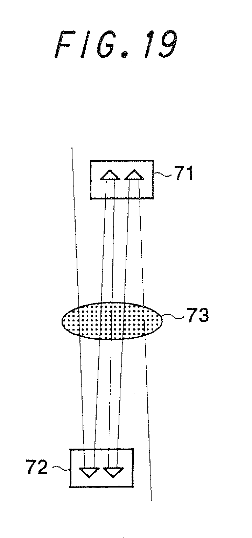

As shown in FIG. 19, the laser beam 55 radiated from the laser irradiation means 66 is repeatedly reflected by means of facing prism means 71 and 72 such that the thus-reflected laser beams do not overlap one another within an ionization zone 73, and a sample introduced into the zone 73 is irradiated with the laser beams. The prism means 71 incorporates a plurality of prisms, but no particular limitations are imposed on the prism means.

Since a plurality of pulse laser beams do not simultaneously impinge on the same portion of the introduced sample, decomposition of the sample is prevented. In addition, efficiency in ionization of the sample through laser irradiation is enhanced.

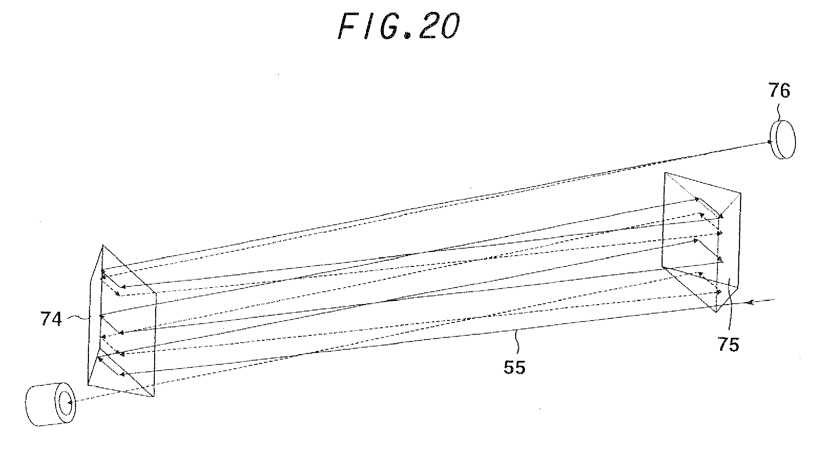

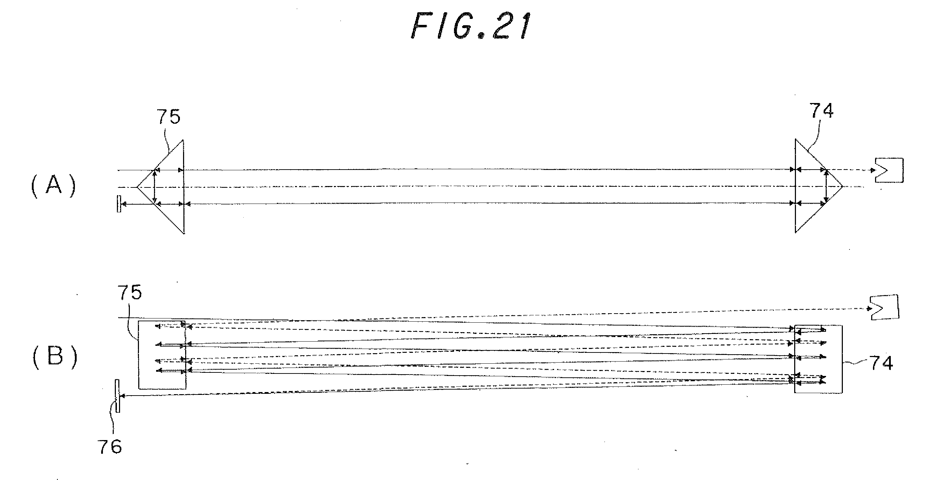

FIGs. 20 and 21 schematically show an optical apparatus for introducing laser beams so as to prevent overlapping of the laser beams. FIG. 20 is a perspective view of the optical apparatus; FIG. 21 (A) is a side view of the apparatus; and FIG. 21 (B) is a plan view of the apparatus.

As shown in FIGs. 20 and 21, the optical apparatus includes prisms 74 and 75 which face each other. When the incident angle of the laser beam 55 is regulated, the reflected laser beams do not overlap one another. In the present embodiment, since a reflection mirror 76 is provided, a sample is repeatedly excited by the reflected laser beams.

In the case where laser irradiation is performed within an ionization zone of 6 mm, when a laser beam of 1 mJ is reflected 10 times by means of the aforementioned prisms, the same effect is obtained as in the case where a laser beam of 10 mJ is employed. Therefore, costs required for the measuring apparatus can be reduced.

In the case where a laser beam is repeatedly reflected, when the thus-reflected laser beams overlap one another, molecules of a measurement target that have been ionized by a laser beam are irradiated again with another laser beam, thereby promoting decomposition of the molecules.

In the present invention, a laser beam is repeatedly reflected such that the thus-reflected laser beams do not overlap one another.

In order to repeatedly reflect a laser beam such that the thus-reflected laser beams do not overlap one another, for example, a technique as shown in FIG. 19 is employed; i.e., a technique employing a plurality of prisms and a refection mirror. Alternatively, there may be employed a technique in which an incident laser beam is regulated by use of a pair of facing prisms. Any technique may be employed in the present invention, so long as a laser beam can be repeatedly reflected such that the thus-reflected laser beams do not overlap one another.

In the case where organic halogenated substances (e.g., PCBs) are subjected to analysis, in order to enhance ionization efficiency of low-chlorine PCBs (i.e., PCBs having 2 to 4 chlorine atoms), the pulse width of an incident laser beam is increased. Meanwhile, in order to enhance ionization efficiency of high-chlorine PCBs (i.e., PCBs having 5 to 7 chlorine atoms), the pulse width of an incident laser beam is lowered.

Through use of two types of laser beams having different pulse widths, the concentrations of low-chlorine PCBs and high-chlorine PCBs can be measured simultaneously.

In the aforementioned embodiments, PCBs are chosen as the measurement targets. However, the present invention is not limited to measurement of PCBs; the present invention can also be applied to measurement of dioxins or environmental hormones contained in waste liquid discharged from incinerators such as a garbage incinerator or from combustion equipment such as a boiler.

### [Eighth embodiment]

Next will be described a system for monitoring a gas in PCB detoxification treatment equipment incorporating the apparatus of the present invention.

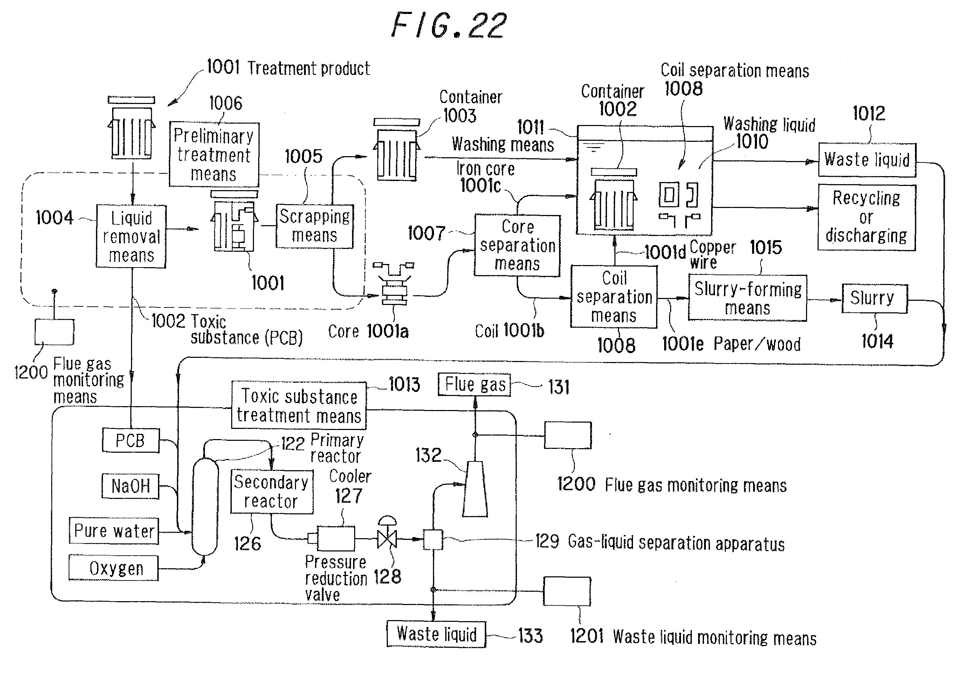

A toxic substance treatment system according to the present embodiment is employed for detoxifying a product to be treated (hereinafter may be referred to as "treatment product") to which a toxic organic halogenated substance (e.g., a PCB) adheres, a treatment product containing such a substance, or a treatment product in which such a substance is stored. As shown in FIG. 22, the treatment system includes preliminary treatment means 1006 including either or both of removal means 1004 for removing a toxic substance 1002 from a container 1003 (i.e., treatment product 1001) containing the toxic substance 1002 (e.g., PCBs) and scrapping means 1005 for scrapping the treatment product 1001 into constitutive members 1001a, 1001b, etc.; core separation means 1007 for separating a core 1001a which - is a constitutive member of the treatment product which has undergone treatment in the preliminary treatment means 1006 - into a coil 1001b and an iron core 1001c; coil separation means 1008 for separating the above-separated coil 1001b into copper wire 1001d and paper/wood 1001e; washing means 1011 for washing, with a washing liquid 1010, the iron core 1001c which has been separated in the core separation means 1007, the metallic container 1003 (including a container main body and a lid) which has been scrapped in the scrapping means 1005, and the copper wire 1001d which has been separated in the coil separation means 1008; toxic substance decomposition treatment means 1013 for decomposing either or both of waste liquid 1012 discharged from the washing means 1011 and the toxic substance 1002 which has been removed in the preliminary treatment means; waste liquid monitoring means 1201 for measuring the concentration of PCBs contained in waste liquid 133 discharged from the toxic substance decomposition treatment means 101 3 (i.e., PCB treatment equipment); and flue gas monitoring means 1200 for measuring the concentration of PCBs within the preliminary treatment means 1006 for scrapping the treatment product and the concentration of PCBs contained in flue gas 131 discharged from the toxic substance decomposition treatment means 1013.

When the aforementioned toxic substance is in the form of liquid, the toxic substance is fed directly into the toxic substance decomposition treatment means 1013, and the substance is detoxified. Constitutive members of the container in which the toxic substance has been stored are also detoxified.

The flue gas discharged from the treatment equipment is caused to pass through an activated carbon filter, and the concentration of PCBs contained in the resultant flue gas is measured by means of the flue gas monitoring means 1200, thereby confirming that the concentration of the PCBs is equal to or lower than a PCB discharge standard.

The concentration of PCBs in the environment outside the treatment equipment, as well as that within the treatment equipment, may be monitored by means of the flue gas monitoring means 1200.

The aforementioned toxic substance treatment means 1013 may be the hydrothermal oxidation-decomposition means shown in FIG. 32, supercritical water oxidation means, or batch-type hydrothermal oxidation-decomposition means.

Toxic substances which cause environmental pollution are detoxified by means of the treatment system of the present invention. Examples of such toxic substances include, but are not limited to, PCBs, vinyl chloride sheets, toxic waste paints, waste fuels, toxic chemicals, waste resins, and untreated explosives.

Examples of the treatment product which is treated by means of the system of the present invention include, but are not limited to, transformers and capacitors containing PCBs serving as insulating oil, and containers in which toxic substances such as paints are stored.

Conventionally, PCBs have been employed in ballasts for fluorescent lamps, and therefore, such ballasts must be detoxified. Since such a ballast has a small volume, the ballast can be detoxified by introducing the ballast directly into the separation means 1007 without performing preliminary treatment.

When the aforementioned toxic substance is in the form of liquid, the toxic substance is fed directly into the toxic substance decomposition treatment means 1013, and the substance is detoxified. The structural members of the container in which the toxic substance has been stored are also detoxified. The concentration of PCBs contained in the thus-detoxified liquid must be confirmed to be equal to or lower than 3 ppb (PCB discharge standard).

Components of the toxic substance treatment means 1013 that are similar to those of the apparatus shown in FIG. 32 are denoted by common reference numerals, and repeated descriptions thereof are omitted.

The flue gas monitoring means 1200 of the present embodiment employs the measuring system 61 including the measuring apparatus 50 shown in FIG. 3, and monitors the concentration of PCBs contained in the flue gas 131 which has been discharged from the treatment means 1013 and cleaned by means of activated carbon.

The waste liquid monitoring means 1201 of the present embodiment employs the measuring system 61 including the measuring apparatus 50 shown in FIG. 3, and monitors the concentration of PCBs contained in the waste liquid 133 which has been discharged from the treatment means 1013 and cleaned by means of activated carbon.

When the aforementioned measuring system is provided, the PCB concentration can be monitored quickly and efficiently. As a result, decomposition treatment can be performed while monitoring for proper performance of treatment processes, thereby taking environment-conscious measures.

Through use of the aforementioned measuring apparatus, PCB analysis can be performed at predetermined intervals, thereby monitoring whether or not the PCB concentration is equal to or lower than a PCB discharge standard. Therefore, in case of an emergency; for example, in the case where the PCB concentration exceeds the discharge standard, flue gas is further cleaned by use of, for example, activated carbon, and operation procedures are reviewed, thereby preventing pollution of the environment outside the treatment system.

### [Ninth embodiment]

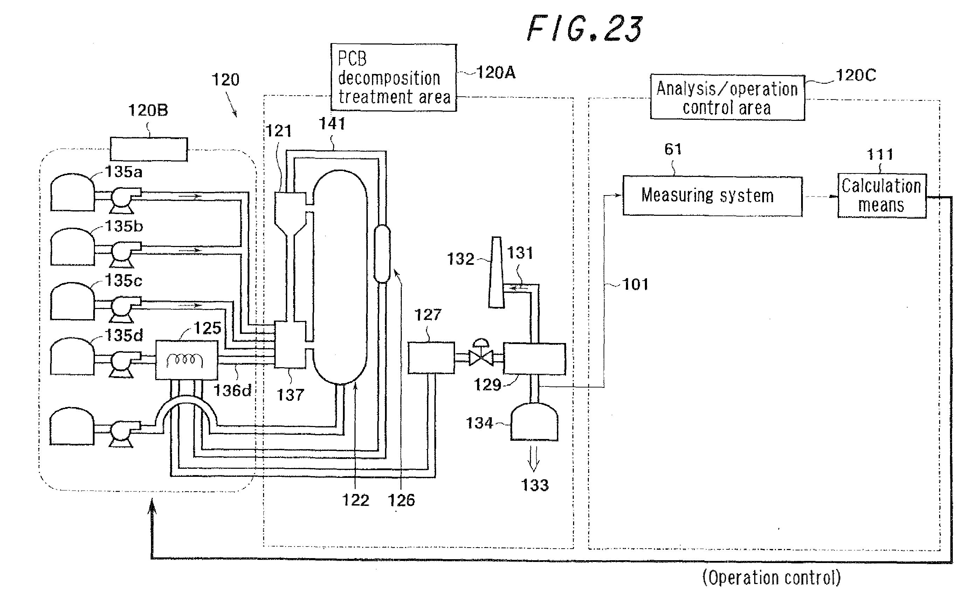

FIG. 23 is a schematic representation showing an organic halogenated substance decomposition treatment system.

The organic halogenated substance decomposition treatment system of the present embodiment will be described by taking, as an example, decomposition treatment of PCBs.

As shown in FIG. 23, the organic halogenated substance decomposition treatment system of the present embodiment includes a hydrothermal oxidation-decomposition apparatus 120 including a heated, pressurized reactor in which PCBs are decomposed into, for example, sodium chloride (NaCl) and carbon dioxide (CO₂) through dechlorination and oxidation-decomposition in the presence of sodium carbonate (Na₂CO₃); and a measuring system 61 for measuring the concentration profiles of PCBs and/or PCB decomposition products remaining in waste liquid 133 discharged from a gas-liquid separation apparatus 129, the measuring system 61 employing laser-type time-of-flight mass spectroscopy.

The measuring system 61 may be any one of the measuring apparatuses according to the first through seventh embodiments.

As shown in FIG. 23, the hydrothermal oxidation-decomposition apparatus 120 of the organic halogenated substance decomposition treatment system includes a PCB decomposition treatment area 120A and a feed area 120B. The hydrothermal oxidation-decomposition apparatus 120 may be the hydrothermal oxidation-decomposition treatment means shown in FIG. 32. However, no particular limitations are imposed on the hydrothermal oxidation-decomposition apparatus, so long as the apparatus enables decomposition of PCBs in the presence of sodium carbonate (Na₂CO₃).

As shown in FIG. 23, the organic halogenated substance (PCBs) decomposition treatment system includes an analysis-operation control area 120C. In the analysis-operation control area 120C, the concentration profiles of PCBs and/or PCB decomposition products remaining in the waste liquid 133 are measured by means of the measuring system 61, and the thus-measured concentration profiles are subjected to calculation processing by calculation means 111, thereby optimizing conditions for feedback control of the hydrothermal oxidation-decomposition apparatus (PCB decomposition treatment apparatus) 120.

Examples of PCBs which are detected by means of the aforementioned apparatus include low-chlorine PCBs such as 1-Cl PCB (monochlorobiphenyl), 2-C1 PCB (dichlorobiphenyl), and 3-Cl PCB (trichlorobiphenyl); and high-chlorine PCBs such as 4-Cl PCB (tetrachlorobiphenyl) and 5-Cl PCB (pentachlorobiphenyl). Such PCBs are detected and subjected to calculation processing.

Examples of the treatment products which are treated by the system of the present invention include, but are not limited to, PCB-containing insulating oils (a variety of oils containing PCBs of low to high concentration) which have been employed in, for example, transformers and capacitors; and PCB-containing paints.

PCBs are decomposed into a variety of products. Examples of such PCB decomposition products include dichlorobenzenes, phthalates, volatile organic compounds, phenol, biphenyl, derivatives of benzene and biphenyl, aldehydes, organic acids, and aromatic hydrocarbons. Specific examples of such PCB decomposition products are described below, but the decomposition products are not limited to these examples.

Examples of dichlorobenzenes include *o*-dichlorobenzene and *p*-dichlorobenzene.

Examples of phthalates include dimethyl phthalate, diethyl phthalate, dibutyl phthalate, and di-2-ethylhexyl phthalate.

Examples of volatile organic compounds (VOCs) include 1,1-dichloroethylene, dichloromethane, *trans*-1,2-dichloroethylene, *cis*-1,2-dichloroethylene, chloroform, 1,1,1-trichloroethane, carbon tetrachloride, benzene, 1,2-dichloroethane, trichloroethylene, 1,2-dichloropropane, dichlorobromomethane, *cis*-1,3-dichloropropene, toluene, *trans*-1,3-dichloropropene, 1,1,2-trichloroethane, tetrachloroethylene, dibromochloromethane, *p*-xylene, *m*-xylene, o-xylene, bromoform, and *p*-diohlorobenzene.

Examples of alkylbenzenes include ethylbenzene, 1,3,5-trimethylbenzene, 1,2,9-trimethylbenzene, sec-butylbenzene, iso-butylbenzene, and n-butylbenzene.

Examples of phenol products include phenol, 2-methylphenol, 4-methylphenol, 2,6-dimethylphenol, 2-ethylphenol, 2,5-dimethylphenol, 3-ethylphenol, 2,3-dimethylphenol, 3,4-dimethylphenol, 2,4,6-trimethylphenol, 2, 3, 6- trimethylphenol, 2,3,5-trimethylphenol, and 4-nonylphenol.

Examples of derivatives of benzene and biphenyl include a styrene monomer, α-methylstyrene, benzyl alcohol, acetophenone, 4'-ethylacetophenone, 2-methylnaphthalene, biphenyl, 1,3-diacetylbenzene, dibenzofuran, fluorene, benzophenone, and xanthone.

Examples of aldehydes include formaldehyde, acetaldehyde, and benzaldehyde.

Examples of organic acids include formic acid, acetic acid, and lactic acid.

Of the aforementioned dichlorobenzenes, *p-*dichlorobenzene or *o*-dichlorobenzene is particularly preferably the object of detection; i.e., the concentration profile of one of these substances is obtained.

Of the aforementioned phthalates, dimethyl phthalate is particularly preferably the object of detection; i.e., the concentration profile of the phthalate is obtained.

Of the aforementioned volatile organic compounds (VOCs), benzene or toluene is particularly preferably the object of detection; i.e., the concentration profile of one of these substances is obtained.

Of the aforementioned alkylbenzenes, ethylbenzene, 1,3,5-trimethylbenzene, or 1,2,4-trimethylbenzene is particularly preferably the object of detection; i.e., the concentration profile of one of these substances is obtained.

Of the aforementioned phenol products, phenol, 2-methylphenol, 4-methylphenol, or 4-nonylphenol is particularly preferably the object of detection; i.e., the concentration profile of one of these substances is obtained.

Of biphenyl products, monochlorobiphenyl, dichlorophenyl, or trichlorobiphenyl is particularly preferably the object of detection; i.e., the concentration profile of one of these substances is obtained.

Of derivatives of benzene and biphenyl, benzyl alcohol, acetophenone, dibenzofuran, or benzophenone is particularly preferably the object of detection; i.e., the concentration profile of one of these substances is obtained.

Of aldehydes, formaldehyde, acetaldehyde, or benzaldehyde is particularly preferably the object of detection; i.e., the concentration profile of one of these substances is obtained.

PCBs and/or PCB decomposition products are detected by means of the detector 59 of the aforementioned measuring apparatus.

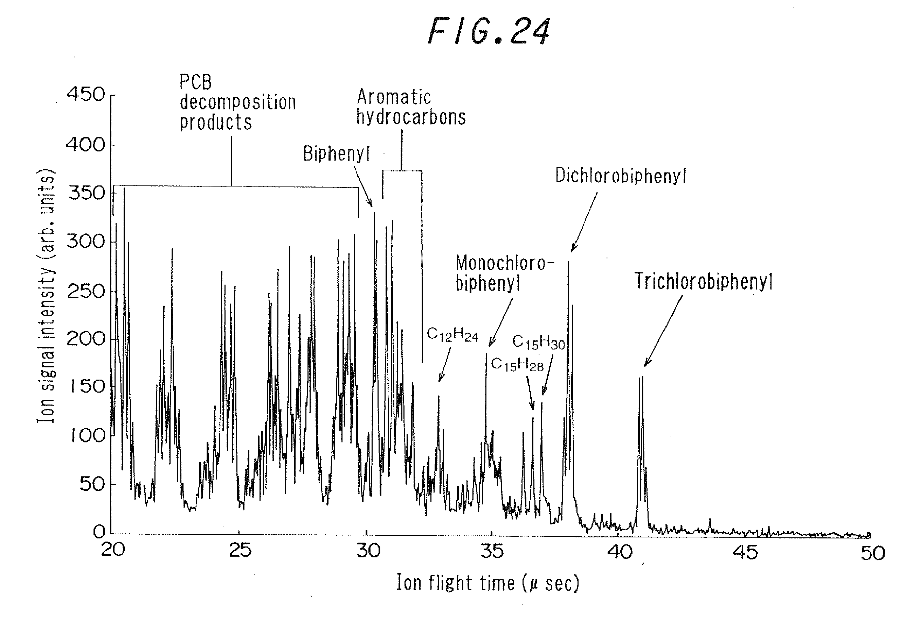

FIG. 24 shows concentration profiles of the thus-detected PCB decomposition products.

FIG. 24 shows concentration profiles of PCB decomposition products contained in the waste liquid 133 obtained through decomposition of PCBs by means of the aforementioned hydrothermal decomposition apparatus 120. The decomposition products include biphenyl, aromatic hydrocarbons, monochlorobiphenyl, dichlorobiphenyl, trichlorobiphenyl, and hydrocarbons such as C₁₂H₂₄, C₁₅H_{28,} and C₁₅H₃₀.

As shown in FIG. 24, PCBs are not detected.

### [Tenth embodiment]

When the aforementioned monitoring system is operated, analysis samples must be collected from a plurality of sampling points. An example of multi-point measurement will now be described in connection with the present embodiment.

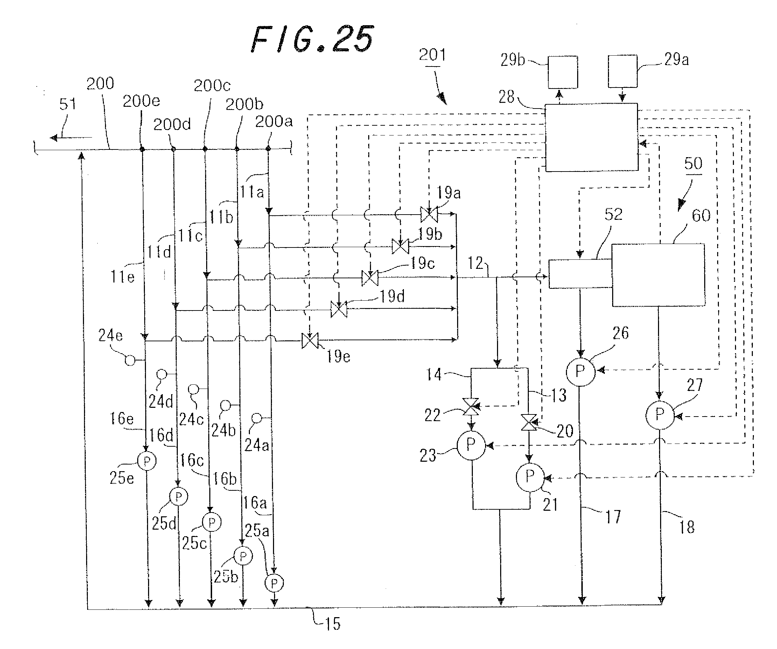

FIG. 25 is a schematic representation showing the overall structure of an organic trace component measuring apparatus according to the present embodiment.

As shown in FIG. 25, first ends of sampling pipes 11a through 11e for sampling flue gas 51 are connected to five sampling points 200a through 200e provided on a flue gas path 200 through which the flue gas 51 passes. Second ends of these sampling pipes 11a through 11e are connected to a first end of a combining pipe 12. A second end of the combining pipe 12 is connected to the sample introduction means of the detection apparatus 50 shown in FIG. 1. A first end of a suction pipe 13 and a first end of an discharge pipe 14 are connected to the combining pipe 12.

A second end of the suction pipe 13 and a second end of the discharge pipe 14 are connected to an discharge pipe 15 connected to the flue gas path 200. The sampling pipes 11a through 11e are connected to the discharge pipe 15 via branch pipes 16a through 16e. The vacuum chamber 52 of the aforementioned detection apparatus 50 is connected to the discharge pipe 15 via an discharge pipe 17. The time-of-flight mass spectrometer 60 of the detection -apparatus 50 is connected to the discharge pipe 15 via an discharge pipe 18.

Vacuum electromagnetic valves 19a through 19e are provided on the sampling pipes 11a through 11e, respectively. A vacuum electromagnetic valve 20 and a diaphragm pump 21 are provided on the suction pipe 13. A vacuum electromagnetic valve 22 and a rotary scroll pump 23 are provided on the discharge pipe 14. Flowmeters 24a through 24e and diaphragm pumps 25a through 25e are provided on the branch pipes 16a through 16e, respectively. A rotary scroll pump 26 is provided on the discharge pipe 17. A high-vacuum pump 27 is provided on the-discharge pipe 18.

As shown in FIG. 1, the detection apparatus 50 employed in the present embodiment includes the vacuum chamber 52 which is connected to the discharge pipe 17 and which is evacuated to about 10⁻¹ torr; the capillary column 54 (sample introduction means) for continuously introducing the flue gas 51 discharged from the combining pipe 12, as a leakage molecular beam 53, into the vacuum chamber 52; the laser irradiation means 66 for irradiating the leakage molecular beam 53 with the laser beam 55 to thereby perform ionization; the convergence section 56 for converging molecules that have been ionized through laser irradiation, the section including a plurality of ion electrodes; the ion trap 57 for selectively trapping the thus-converged ions; and the time-of-flight mass spectrometer 60, which is connected to the vacuum chamber 52 and to the discharge pipe 18, which is evacuated to a high level of vacuum of about 10⁻⁷ to about 10⁻⁸ torr, and which includes the reflectron 58 for reflecting ions which are emitted from the ion trap 57 at predetermined intervals, and the ion detector 59 for detecting the ions.

The ion detector 59 of the time-of-flight mass spectrometer 60 of the detection apparatus 50 is electrically connected to the input section of a control-operation apparatus 28 provided in a control chamber (not illustrated). The vacuum electromagnetic valves 19a through 19e, 20, and 22, the diaphragm pumps 21, the rotary scroll pumps 23 and 26, the high-vacuum pump 27, and the laser irradiation means 66 of the detection apparatus 50 are electrically connected to the output section of the control-operation apparatus 28. An input apparatus 29a and a display apparatus 29b are connected to the control-operation apparatus 28.

In the present embodiment, the suction pipe 13, the vacuum electromagnetic valve 20, and the diaphragm pump 21 constitute gas suction means; the discharge pipe 14, the vacuum electromagnetic valve 22, and the rotary scroll pump 23 constitute cleanup means; the discharge pipe 15 and the branch pipes 16a through 16e constitute a return pipe; and the flowmeters 24a through 24e and the diaphragm pumps 25a through 25e constitute gas circulation means.

An organic trace component measuring apparatus 201 having the aforementioned structure is operated as follows.

Firstly, the diaphragm pumps 25a through 25e are operated; the flue gas 51 passing through the flue gas path 200 is sampled at the sampling points 200a through 200e and introduced into the sampling pipes 11a through 11e, while the respective flow rates of the flue gas 51 passing through the pipes 11a through 11e are confirmed by means of the flowmeters 24a through 24e; and the flue gas 51 is caused to flow through the branch pipes 16a through 16e and returned to the flue gas path 200 via the discharge pipe 15. During the course of the above procedure, the vacuum electromagnetic valves 19a through 19e, 20, and 22 are closed.

Subsequently, when the control-operation apparatus 28 is operated, the vacuum electromagnetic valves 19a and 20 are opened, and the diaphragm pump 21, the rotary scroll pumps 23 and 26, and the high-vacuum pump 27 are operated.

When the laser irradiation means 66 of the detection apparatus 50 is operated, the flue gas 51 - which has been sampled at the sampling point 200a of the flue gas path 200 and passed through the sampling pipe 11a - is introduced, as the leakage molecular beam 53, into the vacuum chamber 52 via the combining pipe 12 and the capillary column 54 of the detection apparatus 50. The molecular beam 53 is irradiated with the laser beam 55 to thereby perform ionization, ions of interest are trapped within the ion trap 57, and the thus-trapped ions are detected by the ion detector 59 of the mass spectrometer 60. On the basis of signals output from the ion detector 59, the control-operation apparatus 28 calculates the concentration of organic trace components such as toxic substances (e.g., PCBs) contained in the flue gas 51 which has been sampled at the sampling point 200a of the flue gas path 200.

After the concentration of toxic substances contained in the flue gas 51 which has been sampled at the sampling point 200a of the flue gas path 200 is measured as described above, the vacuum electromagnetic valves 19a and 20 are closed and the vacuum electromagnetic valve 22 is opened by means of the control-operation apparatus 28, whereby the flue gas 51 which has been sampled at the sampling point 200a of the flue gas path 200 and which remains in the combining pipe 12 is discharged to the flue gas path 200 via the discharge pipe 15, thereby purging the interior of the combining pipe 12.

After the interior of the combining pipe 12 is purged as described above, the vacuum electromagnetic valve 22 is closed and the vacuum electromagnetic valves 19b and 20 are opened by means of the control-operation apparatus 28, whereby the flue gas 51 which has been sampled at the sampling point 200b of the flue gas path 200 and passed through the sampling pipe 11b is introduced, via the combining pipe 12, into the laser irradiation-ionization zone of the detection apparatus 50. Subsequently, in a manner similar to that described above, the concentration of toxic substances contained in the flue gas 51 which has been sampled at the sampling point 200b of the flue gas path 200 is measured. Thereafter, the vacuum electromagnetic valves 19b and 20 are closed, and the vacuum electromagnetic valve 22 is opened, whereby the flue gas 51 which has been sampled at the sampling point 200b of the flue gas path 200 and which remains in the combining pipe 12 is discharged to the flue gas path 200 via the discharge pipe 15, thereby purging the interior of the combining pipe 12.

Subsequently, each of the vacuum electromagnetic valves 19c through 19e is operated in a manner similar to that described above, to thereby perform analysis of the flue gas 51 which has been sampled at the respective sampling points 200c through 200e of the flue gas path 200. Thereafter, the flue gas 51 is discharged from the combining pipe 12, to thereby purge the interior of the pipe 12.

According to the present embodiment, the concentration of toxic substances contained in the flue gas 51 which has been sampled at the sampling points 200a through 200e of the flue gas path 200 can be measured by means of merely the detection apparatus 50. Therefore, costs required for such measurement can be reduced.

Since the interior of the combining pipe 12 is purged by means of the rotary scroll pump 23 after the flue gas 51 is sampled from each of the sampling points 200a through 200e, the concentration of toxic substances contained in the flue gas 51 sampled at each of the sampling points 200a through 200e can be measured accurately.

In the present embodiment, the vacuum electromagnetic valves 19a through 19e, 20, and 22 are employed. However, instead of such a vacuum electromagnetic valve, for example, an electric ball valve or a bellows valve may be employed.

In the present embodiment, the vacuum electromagnetic valve 20 and the diaphragm pump 21 constitute the gas suction means, and the vacuum electromagnetic valve 22 and the rotary scroll pump 23 constitute the cleanup means. However, when a valve whose opening can be finely regulated is employed in combination with a rotary scroll pump, the gas suction means and the cleanup means can be consolidated.

The detection apparatus employed in the present embodiment may be any of the apparatuses according to the second through eighth embodiments.

### [Eleventh embodiment]

When trace components are continuously measured over a long period of time by means of the aforementioned monitoring system, a correction apparatus must be employed. An example of a correction apparatus will now be described in connection with the present embodiment.

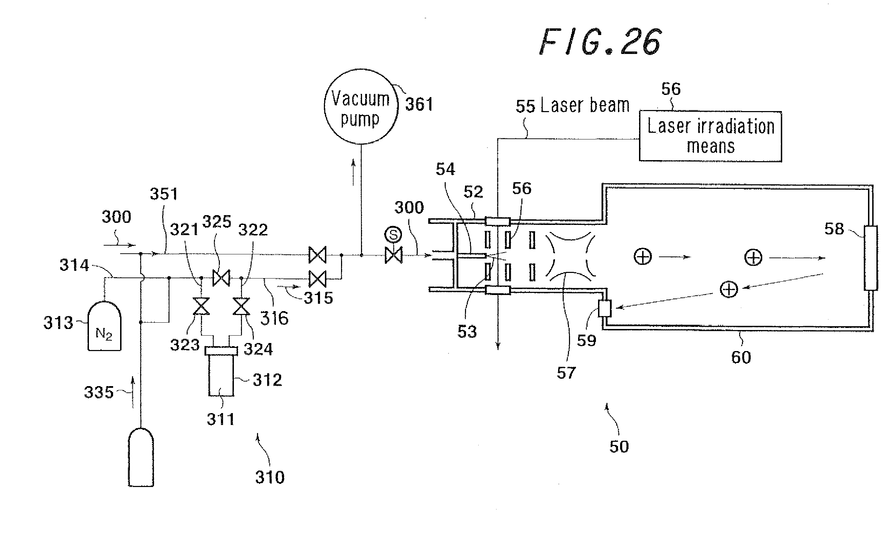

FIG. 26 is a schematic representation showing an organic halogenated substance concentration correction apparatus according to the present embodiment. As shown in FIG. 26, an organic halogenated substance concentration correction apparatus 310 of the present embodiment includes a standard container 312 containing an organic halogenated substance 311 of predetermined concentration; a purge gas feed tube 314 for feeding a purge gas 313 for purging the organic halogenated substance 311 contained in the standard container 312; and a standard gas introduction tube 316 for introducing into a mass spectrometer 50 a standard gas 315 containing the organic halogenated substance 311 of predetermined concentration which is accompanied by the purge gas 313.

The purge gas feed tube 314 has a branched feed line 321 for feeding the purge gas into the standard container. Valves 323 and 324 are provided on the feed line 321 and a gas discharge line 322, respectively. A valve 325 is provided on a path between the purge gas feed tube 314 and the standard gas introduction tube 316 so as to effect opening and closure of the path.

The temperature of the interior of the standard container 312 is maintained at a temperature 5 to 30 degrees higher than the temperature of an atmosphere surrounding the container, by means of temperature-maintaining means (not illustrated), to thereby maintain the saturation concentration of the organic halogenated substance 311 contained in the container.

Table 2 shows the relation between saturation vapor pressure and PCB concentration in the case where the aforementioned organic halogenated substance 311 is PCBs. When the aforementioned halogenated substance is 2- to 4-C1 PCBs, preferably, the temperature of the standard container is maintained at 35°C (i.e., room temperature (25°C) + 10 degrees) by means of a thermostatic bath.

When the aforementioned halogenated substance is 5- to 7-Cl PCBs, preferably, the temperature of the standard container is maintained at 50°C (i.e., room temperature (25°C) + 25 degrees) by means of a thermostatic bath.

**[Table 2]**

| Saturation vapor pressure | PCB concentration |
|---|---|
| 10 mg/Nm³ | 2-Cl PCB ··· 0.2 mg/Nm³ |
| 3 mg/Nm³ | 3-Cl PCB ··· 0.5 mg/Nm³ |
| 0.5 g/Nm³ | 4-Cl PCB ··· 0.05 mg/Nm³ |

Kanechlor KC300 (product of Kanegafuchi Chemical Industry Co., Ltd.), a commercially available PCB product, contains 2-Cl PCB, 3-Cl PCB, and 4-Cl PCB. Kanechlor KC400 (product of Kanegafuchi Chemical Industry Co., Ltd.) contains 3-Cl PCB, 4-Cl PCB, and 5-Cl PCB. Kanechlor K60 (product of Kanegafuchi Chemical Industry Co., Ltd.) contains 5-Cl PCB, 6-C1 PCB, and 7-C1 PCB.

In order to maintain the saturation concentration of PCBs at a predetermined level, a PCB solution prepared by dissolving PCBs in a standard liquid (e.g., n-hexane) is fed into the standard container; the container is subjected to degassing treatment under vacuum for one hour so as to remove n-hexane; and the container is sealed and the temperature of the container is maintained at 100°C for one hour, thereby rendering the concentration of the PCBs uniform within the container.

Temperature-maintaining means 326 (e.g., a heater) is provided on the standard gas introduction tube 316 so as to maintain the temperature of the interior of the tube 316 at 150 ± 20°C, thereby preventing adhesion of an organic halogenated substance to the inner wall of the tube.

Preferably, the distance D between the standard gas introduction tube 316 and the standard container 312 is regulated such that the distance D is about 10 times the inner diameter (φ) of the standard gas introduction tube 316.

In the case where the distance D is short, when the valve 324 is opened, the heated gas enters the standard container. Therefore, in order to avoid such a problem, the distance D is regulated as described above.

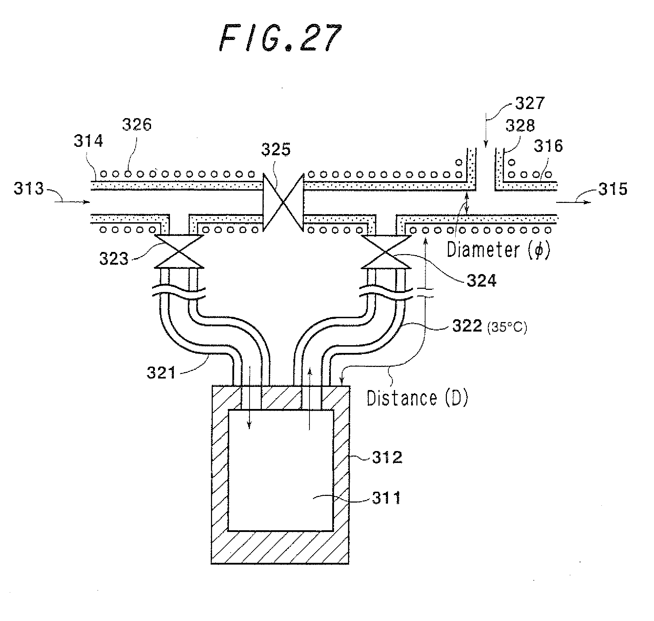

At present, the regulation limit of flue gas discharged from the aforementioned PCB treatment equipment is 0.15 mg/Nm³ (i.e., 15 ppb/V). Therefore, a standard gas containing PCB in an amount 1/5 the above value is employed for concentration correction in the mass spectrometer. As shown in FIG. 27, a dilution gas feed pipe 328 for feeding a dilution gas 327 (e.g., air or nitrogen) is provided on the standard gas introduction tube 316. The standard gas 315 is diluted with the dilution gas 327 so as to attain a predetermined concentration.

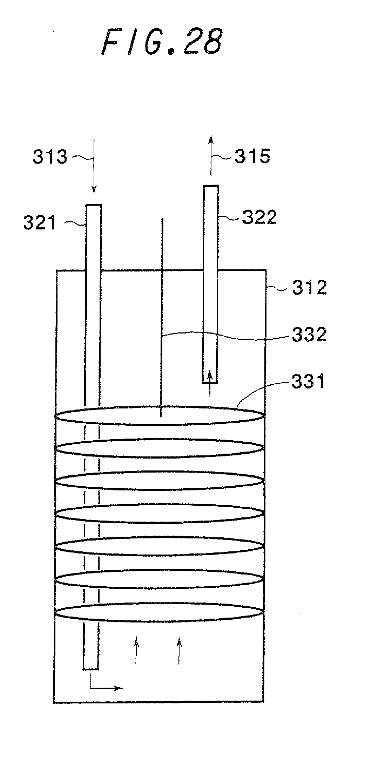

As shown in FIG. 28, the standard container 312 includes a plurality of disks 331, each having a plurality of pores; and the feed line 321 for feeding the purge gas 313 to the bottom of the container 312. The purge gas 313 is introduced to the bottom of the container 312, and then caused to pass through numerous pores of the disks 331, to thereby carry saturated PCBs to the outside of the container.

Thus, a PCB standard sample of uniform concentration can be introduced into the mass spectrometer.

In FIG. 28, reference numeral 332 denotes a thermometer.

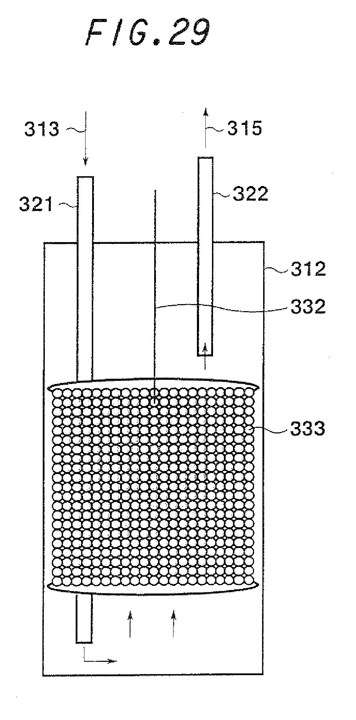

As shown in FIG. 29, instead of employing the disks 331, the standard container may be filled with glass fiber or beads 333.

The inner wall of the standard container 312 is covered with a coating layer formed of, for example, polytetrafluoroethylene or silicon oxide. Such a coating layer is provided in order to prevent PCBs from penetrating into the inner wall of the container.

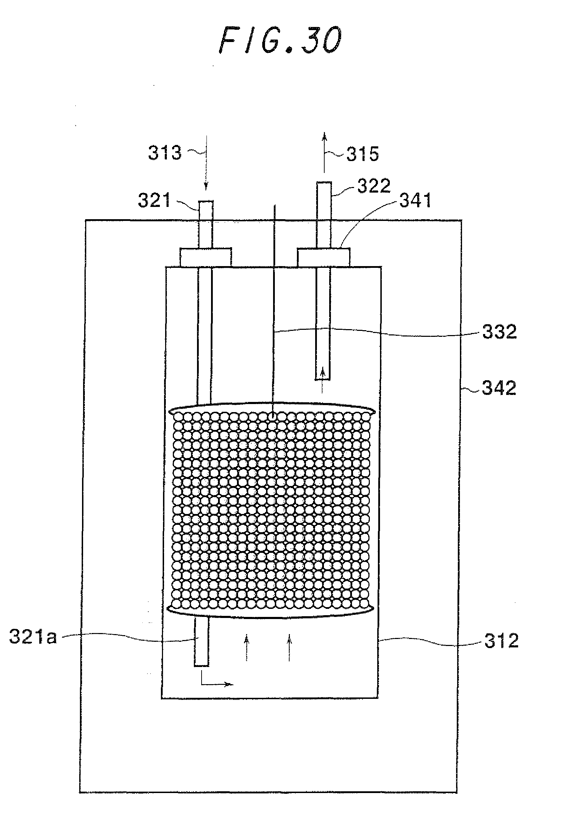

As shown in FIG. 30, the standard container 312 may be of detachable cartridge type having a detachable member 341.

Thus, detachment of the standard container 312 is readily attained, and any type of standard container can be provided.

As shown in FIG. 30, the detachable standard container 312 may be provided in a hermetic container 342. When the container 312 is provided in the hermetic container 342, leakage of PCBs to the outside can be prevented during the course of attachment/detachment of the container 312.

When a detection substance is fed into the standard container 312, and a sensor for detecting the detection substance is provided in the hermetic container 342, improper attachment of the container 312 can be discovered at an early stage.

The aforementioned detection substance is preferably hydrogen among other substances. When the purge gas 313 containing hydrogen in an amount of about some percent is fed into the container 312, and known detection means is provided on the inner wall of the hermetic container 342, leakage of the hydrogen can be detected quickly. As a result, improper attachment of the container 312 can be discovered through detection of the hydrogen (detection substance). The amount of hydrogen in the purge gas may be 100%. However, in consideration of leakage of hydrogen, the amount of hydrogen in the purge gas is preferably 4% (i.e., the lower explosive limit of hydrogen) or less.

When a line 300 for sampling an organic halogenated substance is provided in the hermetic container including the cartridge-type standard container 312, on-line measurement of the concentration of the organic halogenated substance can be performed by means of the measuring system 61 including the measuring apparatus 50 according to any one of the first through seventh embodiments.

In the case where the aforementioned halogenated substance concentration correction apparatus 310 is provided, when the concentration of PCBs in PCB treatment equipment is continuously measured, even if variation in measurement conditions arises in the time-of-flight mass spectrometer 60, such variation can be corrected quickly.

An internal standard gas 35 of predetermined concentration for monitoring (e.g., monochlorobenzene) is fed to the sample introduction tube 51 and to the purge gas feed tube 314. Variation in measurement conditions can be confirmed by monitoring the concentration of the gas 35.

In general, the concentration of PCBs as measured by means of the aforementioned apparatus is nearly equal to zero. Therefore, difficulty is encountered in determining whether the thus-measured PCB concentration is actually zero or the PCB concentration is inaccurately determined to be zero as a result of anomalous operation of the measuring apparatus or stuffing of pipes. However, when the aforementioned monitoring gas is fed, if the intensity of the peak corresponding to the thus-fed monitoring gas varies - although the intensity of the peak is generally found to be constant - anomalous operation of the measuring apparatus or stuffing of pipes can be discovered quickly.

The concentration of the standard gas 315 can be corrected by confirming that the ratio between the intensity of the peak corresponding to the monitoring gas 35 and that of the peak corresponding to the standard gas 315 is constant.

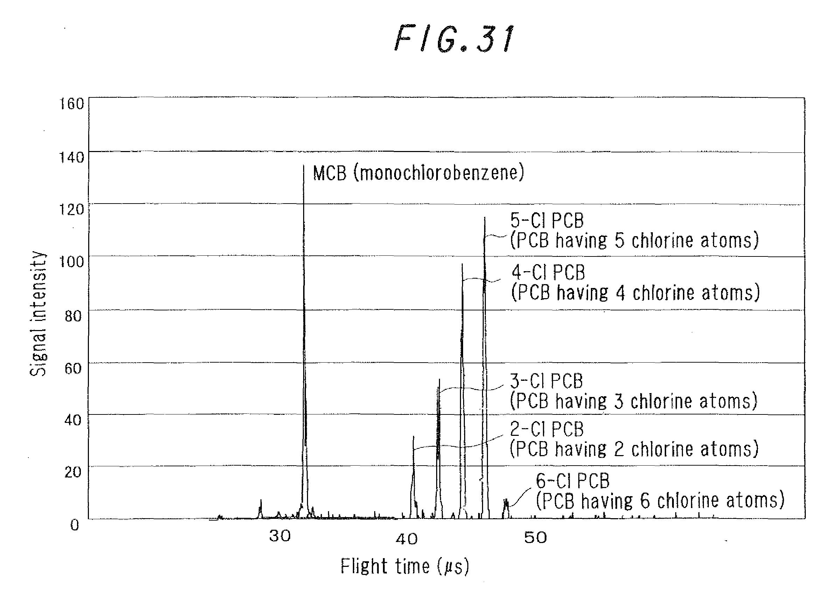

FIG. 31 is a chart showing results of measurement of the standard gas and the monitoring gas.

The measuring apparatus which can be employed in the correction apparatus of the present invention is not limited to the aforementioned measuring apparatus.

In the case where the concentration of PCBs is measured by means of the aforementioned measuring apparatus at predetermined intervals, when the gas for concentration correction is fed from the correction apparatus into the measuring apparatus after elapse of a predetermined period (e.g., one week or 10 days), the measuring apparatus can be operated properly.

### Industrial Applicability

As described above, the present invention provides an apparatus for detecting an organic halogenated substance contained in a gas. The detection apparatus includes sample introduction means for continuously introducing a collected sample into a vacuum chamber; laser irradiation means for irradiating the thus-introduced sample with a laser beam to thereby ionize the sample; a convergence section for converging molecules that have been ionized through laser irradiation; an ion trap for selectively trapping the thus-converged molecules; and a time-of-flight mass spectrometer incorporating an ion detector for detecting ions which are emitted at predetermined intervals. The detection apparatus enables quick analysis of organic halogenated substances such as PCBs and dioxins.

## Claims

1. An organic trace component measuring apparatus comprising:
an organic trace component detection apparatus;
a plurality of sampling pipes for sampling a gas from sampling points provided on a gas path through which the gas passes;
a valve provided on each of the sampling pipes;
a combining pipe for connecting the sampling pipes to the laser irradiation means of the detection apparatus;
gas suction means for circulating the gas, which is connected to the combining pipe; and
cleanup means for discharging to the outside the gas remaining in a portion between the valve provided on each of the sampling pipes and the detection apparatus, the cleanup means being connected to the combining pipe,
wherein said organic trace component detection apparatus comprises
sample introduction means for continuously introducing a collected sample into a vacuum chamber;
laser irradiation means for irradiating the thus-introduced sample with a laser beam to thereby ionize the sample;
a convergence section for converging molecules that have been ionized through laser irradiation;
an ion trap for selectively trapping the thus-converged molecules; and
a time-of-flight mass spectrometer incorporating an ion detector for detecting ions which are emitted at predetermined intervals.

2. An organic trace component measuring apparatus according to claim 1,
which further comprises a return pipe which is provided between the valve and a point at which each of the sampling pipes and the gas path are connected and which is connected to the gas path; and gas circulation means for circulating a gas, which is provided on the return pipe.

3. An organic trace component measuring apparatus according to claim 1,
wherein the gas suction means comprises a diaphragm pump connected to the combining pipe, and a valve provided between the combining pipe and the diaphragm pump.

4. An organic trace component measuring apparatus according to claim 1,
wherein the cleanup means comprises a rotary scroll pump connected to the combining pipe, and a valve provided between the combining pipe and the rotary scroll pump.

5. An organic trace component measuring apparatus according to claim 1,
wherein the valve is any valve selected from among a vacuum electromagnetic valve, an electric ball valve, and a bellows valve.

6. A apparatus for detecting an organic trace component according to claim 1, comprising means for continuously introducing a collected sample into a vacuum chamber; irradiating the thus-introduced sample with a laser beam to thereby ionize the sample; selectively trapping, in an ion trap, molecules ionized through laser irradiation while converging the molecules; and detecting, by use of a time-of-flight mass spectrometer, ions which are emitted from the ion trap at predetermined intervals,
wherein the ion trap comprises a first end cap electrode having a small hole through which the ionized molecules enter, a second end cap electrode having a small hole from which the trapped molecules are emitted, the first and second end cap electrode facing each other, and a high-frequency electrode for applying a high-frequency voltage to the ion trap; the voltage of the first end cap electrode is lower than that of an ion convergence section for converging the ionized molecules, and the voltage of the second end cap electrode is higher than that of the first end cap electrode; and the ionized molecules are trapped under application of the high-frequency voltage while the molecules within the ion trap are selectively decelerated.

7. An apparatus for detecting an organic trace component according to claim 6,
wherein an inert gas is caused to flow within the ion trap, an ionization zone has a vacuum of 1 x 10⁻³ torr, the ion convergence section and the ion trap have a vacuum of 1 x 10⁻⁵ torr, and the time-of-flight mass spectrometer has a vacuum of 1 x 10⁻⁷ torr.

8. An apparatus for detecting an organic trace component according to claim 6,
wherein the gas is a gas in treatment equipment where PCB decomposition treatment has been performed.

9. An apparatus for detecting an organic trace component according to claim 6,
wherein the laser beam radiated from a laser irradiation means is repeatedly reflected such that the thus-reflected laser beams do not overlap one another within the ionization zone.

10. An apparatus for detecting an organic trace component according to claim 9,
wherein the laser beam radiated from the laser irradiation means is repeatedly reflected by use of facing prisms such that the thus-reflected laser beams do not pass through the same path.

11. An organic substance decomposition treatment system comprising a hydrothermal oxidation-decomposition apparatus including a heated and pressurized reactor in which an organic halogenated substance is decomposed into, for example, sodium chloride (NaCl) and carbon dioxide (CO₂) through dechlorination and oxidation-decomposition in the presence of sodium carbonate (Na₂CO₃); an organic trace component detection apparatus as recited in claim 1 for measuring the concentration of a toxic substance and/or a product produced through decomposition of the toxic substance contained in a waste liquid discharged from the hydrothermal oxidation-decomposition apparatus; and operation control means for controlling operation of the hydrothermal oxidation-decomposition apparatus on the basis of measurement results obtained from the organic trace component detection apparatus.

12. An organic substance decomposition treatment system according to claim 11,
wherein the hydrothermal oxidation-decomposition apparatus comprises a cylindrical primary reactor including a cyclone separator; a pressurizing pump for pressurizing oil or an organic solvent, a toxic substance, water (H₂O), and sodium hydroxide (NaOH); a preheater for preliminarily heating the water; a secondary reactor having a spiral pipe; a cooler for cooling a treated liquid discharged from the secondary reactor; gas-liquid separation means for subjecting the treated liquid to gas-liquid separation; and a pressure reduction valve.

13. An organic substance decomposition treatment system according to claim 11,
wherein the operation control means controls at least one selected from among heating of the toxic substance decomposition treatment system, pressurization of the system, the feed amount of a liquid for treating the toxic substance, the feed amount of an oxidizing agent, and the feed amount of sodium hydroxide (NaOH).

14. An organic halogenated substance concentration correction apparatus for correcting the organic trace component detection apparatus as recited in claim 1,
which comprises a standard container containing an organic halogenated substance of predetermined concentration; and a standard gas introduction tube for feeding into the standard container a purge gas for purging the organic halogenated substance, to thereby introduce into a mass spectrometer the organic halogenated substance accompanied by the purge gas.

15. An organic halogenated substance concentration correction apparatus according to claim 14,
which further comprises temperature-maintaining means for maintaining the temperature of the standard container at a temperature 5 to 100 degrees higher than the temperature of an atmosphere surrounding the container.

16. An organic halogenated substance concentration correction apparatus according to claim 14,
which further comprises temperature-maintaining means for maintaining the temperature of the standard gas introduction means at 150°C or higher.

17. An organic halogenated substance concentration correction apparatus according to claim 14,
wherein a disk having a plurality of pores is provided in the standard container.

18. An organic halogenated substance concentration correction apparatus according to claim 14,
wherein the standard container is filled with glass fiber or beads.

19. An organic, halogenated substance concentration correction apparatus according to claim 17,
wherein a feed tube for feeding a purge gas is provided at the bottom of the standard container such that the outlet of the feed tube faces the bottom, and the fed purge gas is discharged from the upper portion of the container.

20. An organic halogenated substance concentration correction apparatus according to claim 14,
wherein the inner wall of the standard container is covered with a coating layer formed of polytetrafluoroethylene or silicon oxide.

21. An organic halogenated substance concentration correction apparatus according to claim 14,
wherein the standard container is removably provided.

22. An organic halogenated substance concentration correction apparatus according to claim 21,
wherein the removable standard container is provided in a hermetic container.

23. An organic halogenated substance concentration correction apparatus according to claim 22,
wherein a detection substance is fed into the standard container, and a sensor for detecting the detection substance is provided in the hermetic container.

24. An organic halogenated substance concentration correction apparatus according to claim 23,
wherein the detection substance is hydrogen.

25. An organic halogenated substance concentration correction apparatus according to claim 14,
wherein the sample contains PCBs.

26. A method for detecting an organic trace component comprising detecting an organic halogenated substance while correcting at predetermined intervals the concentration of the organic halogenated substance by use of the organic halogenated substance concentration correction apparatus as recited in claim 14.

27. A method for detecting an organic trace component according to claim 26,
wherein the sample contains PCBs, and the organic halogenated substance is a PCB.
